# EUROPEAN PATENT APPLICATION

(11) **EP 4 818 213 A1**
(43) Date of publication of application: **07.10.2026**
(21) Application number: 24911267.3
(22) Date of filing: 25.12.2024
(51) Int. Cl.: C07K 19/00, C12N 15/63, C12N 5/10, C12N 15/11, A61K 38/00, A61P 37/02, A61P 35/00

(54) **TIGIT POLYPEPTIDE AND TIGIT/CTLA4 FUSION PROTEIN**

(30) Priority: 29.12.2023 CN 202311873108
(71) Applicant: Shenyang Sunshine Pharmaceutical Co., Ltd., Shenyang, Liaoning 110027 (CN)
(72) Inventor: HUANG, Haomin, Shenyang, Liaoning 110027 (CN); ZHANG, Xuesai, Shenyang, Liaoning 110027 (CN); LOU, Jing, Shenyang, Liaoning 110027 (CN)
(74) Representative: Canzler & Bergmeier Patentanwälte Partnerschaft mbB
(86) International application number: PCT/CN2024/142449
(87) International publication number: WO 2025/140345

(57) **Abstract**

Provided in the present application are a TIGIT polypeptide and a TIGIT/CTLA4 fusion protein. The TIGIT polypeptide of the present application has high affinity, the TIGIT/CTLA4 fusion protein of the present application can simultaneously block the CD226/CD155 signaling pathway and the CD80/CD86-CD28 signaling pathway, and the polypeptide and the fusion protein have clinical application prospects in treating immune system-related diseases.

## Description

### CROSS-REFERENCE TO RELATED APPLICATIONS

This application claims the priority of Chinese Patent Application No. 202311873108.7, filed with the China National Intellectual Property Administration on December 29, 2023, entitled "TIGIT POLYPEPTIDE AND TIGIT/CTLA4 FUSION PROTEIN", the entire content of which is incorporated herein by reference.

### TECHNICAL FIELD

The present application relates to the field of recombinant protein technology, and specifically, to a TIGIT polypeptide and a TIGIT/CTLA4 fusion protein.

### BACKGROUND

CTLA-4, which stands for cytotoxic T lymphocyte-associated antigen-4 (CTLA-4), also known as CD152, is a transmembrane receptor primarily expressed on activated T cells. It is homologous to the costimulatory protein receptor CD28 on the T cell surface. Both can bind to CD80/CD86 (also known as B7-1 and B7-2) on antigen-presenting cells (APCs). CTLA-4 transmits inhibitory signals to T cells, while CD28 transmits stimulatory signals. Studies have found that CTLA-4 captures B7-1 and B7-2 on the APC membrane and then undergoes endocytosis, thereby preventing these ligands from activating the CD28 signaling pathway. CTLA4 located on the cell surface exists as a dimer. CTLA4 exhibits high endocytic activity, and only 10% of CTLA4 is located on the cell surface. Furthermore, the ligands CD80/CD86 are mainly expressed on B cells, DC cells, macrophages, and monocytes. CD86 exists as a monomer, while CD80 exists as a dimer. Therefore, CD80 has a stronger affinity for both CD28 and CTLA4 than CD86.

TIGIT (T cell Ig and ITIM domain) is a member of the poliovirus receptor (PVR)/Nectin family. The transmembrane protein TIGIT consists of an extracellular immunoglobulin variable domain (IgV), a type 1 transmembrane domain, and an intracellular domain containing a classical immunoreceptor tyrosine-based inhibitory motif (ITIM) and an immunoglobulin tyrosine tail (ITT) motif. TIGIT is highly expressed on lymphocytes, primarily on CD4⁺ regulatory T cells and follicular helper T cells, CD8⁺ effector T cells, and NK cells. Natural ligands for TIGIT include CD155, CD112, and CD113, with high affinity for CD155 and low affinity for CD112 and CD113.The signaling pathways of TIGIT include inhibiting cytokine production and cytotoxicity of NK cells, inhibiting the activation and proliferation of T cells, and enhancing the proliferation and suppressive activity of Treg cells. Additionally, studies have shown that TIGIT can induce tolerogenic dendritic cells.

Rheumatoid arthritis (RA) is an autoimmune disease involving multiple genes and multiple immune cells. Specifically, in genetically susceptible individuals, host and environmental factors can lead to the activation of pathogenic T cells and a subsequent series of inflammatory responses. Cytokines participate in coordinating immune responses as well as the recruitment and activation of effector cells, leading to inflammation while also causing local tissue damage in the patient's joints and involvement of extra-articular organs. Patients with rheumatoid arthritis are generally treated with disease-modifying antirheumatic drugs (DMARDs). When a patient has an inadequate response to chemical DMARDs, concomitant treatment with biological disease-modifying antirheumatic drugs (bDMARDs) needs to be considered. The targets of the action of biological disease-modifying antirheumatic drugs are primarily inflammatory factors, CD20-positive B cells, or the regulation of T cell co-stimulatory signals.CTLA4 fusion proteins can specifically inhibit the T cell CD28-CD80/CD86 co-stimulatory signaling pathway, and can also inhibit other immune cell populations positive for CD80/CD86, including activated B cells, macrophages, osteoclasts, and endothelial cells, making it a target with great development potential among biological disease-modifying antirheumatic drugs. Weigong Zhao et al. found in studies on RA model mice that the expression level of TIGIT is negatively correlated with the progression of rheumatoid arthritis. The expression level of TIGIT on CD4-positive T cells in the synovial fluid of severely affected joints was significantly lower than that in milder cases, while overexpression of TIGIT in CD4-positive cells significantly reduced the proliferation level of cells and the secretion of cytokines IFN-γ and IL-17.First, mechanistically, the binding of TIGIT expressed on the surface of T cells to the CD155 receptor on the surface of DC cells induces an inhibitory signaling pathway in the T cells themselves, and also induces the generation of immunosuppressive DCs through the expression of IL-10.Furthermore, TIGIT has a higher affinity for CD155 than CD226, thereby disrupting the dimerization of CD226 and CD226-regulated T cell activation. Therefore, mechanistically, TIGIT is also one of the highly promising targets for biological disease-modifying antirheumatic drugs.

Type 1 diabetes (T1D) is an autoimmune disease primarily mediated by T cells, where an overactive immune system attacks the insulin-producing beta cells, leading to insufficient insulin secretion. Although the discovery and large-scale production of insulin have made T1D no longer a terminal illness, many patients still develop complications as the disease progresses, including cardiovascular disease, retinopathy, neuropathy, and nephropathy. From a pathogenesis perspective, the development of T1D primarily involves CD4⁺ T cells and CD8⁺ T cells.CD4⁺ T cells are involved because their HLA class II haplotypes are strongly associated with T1D.In addition to finding direct evidence of CD4⁺ T cell pathogenicity in the NOD (non-obese diabetic) mouse model, researchers have found that CD4⁺ T cells carrying islet antigens (Ag) in the blood and islets of T1D patients assist B cells, T cells, and the cytokines they produce in activating macrophages and islet-specific CD8⁺ T cells. Pathogenic CD8⁺ T cells react with various overlapping islet antigens (including insulin, IGRP, ZnT8, proinsulin) and some neoantigens (such as hybrid peptides and corresponding post-translational modification products), triggering the destruction of pancreatic beta cells. Furthermore, other regulatory T cell subsets, such as Tr1, Th3, and CD8⁺ T suppressor cells, have also been reported to be involved in the development of T1D.Fumitaka Haseda et al. found that the expression of CTLA4 in CD4-positive helper T cells of T1D patients was significantly reduced. In the autoimmune-prone non-obese diabetic mouse model simulating T1D, the expression of CTLA4 was significantly reduced, and the deficiency of CTLA4 led to significant immune system dysregulation, manifested as overactivation of CD4⁺ T cells. The co-inhibitory signaling pathway of CTLA4 for CD4⁺ T cells plays an important role in this process. Sven Brode et al. found that the number and function of CD4⁺CD25⁺ Tregs were reduced in the NOD mouse model, which also indicates that low expression of CTLA4 affects the proliferation and function of CD4⁺CD25⁺ Tregs. For the treatment of T1D, the most direct goal is to ensure the normal function of beta cells and improve the immune tolerance and unresponsiveness of T cells. Similar to the CD28/CTLA4/CD80/CD86 pathway, CD226 can activate CD4⁺, CD8⁺, and NK cells by binding to CD155, while TIGIT has the opposite effect. Therefore, although there are currently few studies on the specific mechanisms of CD226 and TIGIT in the pathogenesis of T1D, it is certain that the CD226/TIGIT axis, which respectively activates and inhibits T cell activation, is crucial for regulating T cell immune tolerance.

The complex mechanisms underlying immune system diseases involve the activation of multiple signaling pathways and diverse immune cell activities, thereby limiting the efficacy of conventional single-target drugs. This highlights the critical need for developing novel dual-target therapeutic agents.

### SUMMARY OF THE INVENTION

An object of the present application is to provide a TIGIT polypeptide variant and a TIGIT/CTLA4 fusion protein thereof. The further objects of the present application are to provide a nucleic acid molecule encoding the polypeptide variant and the fusion protein thereof; to provide an expression vector comprising the nucleic acid molecule; to provide a host cell comprising the expression vector; to provide a method for preparing the polypeptide variant and the fusion protein thereof; to provide a pharmaceutical composition comprising the polypeptide variant and the fusion protein thereof; and to provide a medical use of the polypeptide variant and the fusion protein thereof or the pharmaceutical composition for preventing or treating immune-related diseases.

To achieve the above objects, the present application provides the following technical solutions:
A first aspect of the present application provides a TIGIT polypeptide variant, comprising a TIGIT extracellular domain or a functional fragment thereof,
wherein the variant comprises at least one amino acid mutation relative to the amino acid sequence of SEQ ID NO: 3, and the mutation position is selected from one or more of the following group: residues I42, Q56, N70, L73, G74, Y113.

A second aspect of the present application provides a fusion protein, comprising the TIGIT polypeptide variant.

A third aspect of the present application provides an expression vector, comprising the nucleic acid.

A fourth aspect of the present application provides a host cell, comprising the expression vector or expressing the polypeptide variant or fusion protein.

A fifth aspect of the present application provides a method for preparing the polypeptide variant or fusion protein, comprising the steps of: synthesizing the polypeptide variant or fusion protein, and/or, culturing the host cell under conditions that express the polypeptide variant or fusion protein.

A sixth aspect of the present application provides a pharmaceutical composition, comprising the polypeptide variant, the fusion protein, and optionally a pharmaceutically acceptable excipient or carrier.

A seventh aspect of the present application provides a use of the polypeptide variant, the fusion protein, or the pharmaceutical composition in the preparation of a medicament for preventing or treating an immune system disease or tumor.

An eighth aspect of the present application provides a method for preventing or treating an immune system disease or tumor, comprising administering a pharmaceutically effective amount of the polypeptide variant, the fusion protein, or the pharmaceutical composition to a subject.

The objectives of the present application also include providing a

TIGIT/CTLA4 fusion protein; providing a nucleic acid molecule encoding the fusion protein; providing an expression vector comprising the nucleic acid molecule; providing a host cell comprising the expression vector; providing a method for preparing the fusion protein; providing a pharmaceutical composition comprising the fusion protein; and providing a medical use of the fusion protein or the pharmaceutical composition for preventing or treating an immune system-related disease.

To achieve the above objects, the present application provides the following technical solutions:
A ninth aspect of the present application provides a fusion protein, comprising a first region and a second region, or comprising a first region, a second region, and a third region; the first region is a TIGIT polypeptide or a variant thereof, the second region is a CTLA4 polypeptide or a variant thereof, and the third region is an Fc polypeptide or a variant thereof; the TIGIT or CTLA4 comprises an extracellular domain or a functional fragment thereof.

A tenth aspect of the present application provides a nucleic acid encoding the fusion protein.

An eleventh aspect of the present application provides an expression vector comprising the nucleic acid.

A twelfth aspect of the present application provides a host cell comprising the expression vector or expressing the fusion protein.

A thirteenth aspect of the present application provides a method for preparing the fusion protein, comprising the steps of: synthesizing the fusion protein, and/or culturing the host cell under conditions that express the fusion protein. A fourteenth aspect of the present application provides a pharmaceutical composition comprising the fusion protein and optionally a pharmaceutically acceptable excipient or carrier.

A fifteenth aspect of the present application provides use of the fusion protein or the pharmaceutical composition in the preparation of a medicament for preventing or treating an immune system disease or tumor.

A sixteenth aspect of the present application provides a method for preventing or treating an immune system disease or tumor, comprising administering a pharmaceutically effective amount of the fusion protein or the pharmaceutical composition to a subject.

### Brief Description of the Drawings

FIG. 1 shows an ELISA detection diagram of the affinity of a CTLA4 fusion protein for CD86.
FIG. 2 shows an ELISA detection diagram of the affinity of a TIGIT fusion protein for CD155.
FIG. 3 shows a configuration diagram of a bispecific fusion protein.
FIG. 4 shows an ELISA detection diagram of the affinity of a bispecific fusion protein for CD86.
FIG. 5 shows an ELISA detection diagram of the affinity of a bispecific fusion protein for CD155.
FIG. 6 shows an ELISA detection diagram of the affinity of a first-round mutant TIGIT fusion protein for CD155.
FIG. 7 shows an ELISA detection diagram of the affinity of a second-round mutant TIGIT fusion protein for CD155.
FIG. 8 shows a binding kinetics detection diagram of a TIGIT fusion protein for CD155.
FIG. 9 shows a detection diagram of the blockade of the CD226/CD155 signaling pathway by a TIGIT fusion protein.
FIG. 10 shows a detection diagram of the blockade of the CD226/CD155 signaling pathway by a bispecific fusion protein.
FIG. 11 shows a detection diagram of the blockade of the CD80/CD86-CD28 signaling pathway by a bispecific fusion protein.
FIG. 12 shows a detection diagram of the inhibition of CD4⁺ T cell activation by a bispecific fusion protein.
FIG. 13 shows a detection diagram of the inhibition of CD8⁺ T cell activation by a bispecific fusion protein.
FIG. 14 shows the arthritis scores of the four limbs in a collagen-induced arthritis mouse model inhibited by a fusion protein.
FIG. 15 shows the measurement data of bilateral hind paw swelling in a collagen-induced arthritis mouse model inhibited by a fusion protein.

### Detailed Description

The embodiments of the present disclosure are described in detail below. The embodiments described below are exemplary and are intended only to explain the present disclosure, and should not be construed as limiting the present disclosure. The technical solutions in the embodiments of the present disclosure will be clearly and completely described below. Obviously, the described embodiments are only some, but not all, of the embodiments of the present disclosure. Based on the embodiments in the present disclosure, all other embodiments obtained by a person of ordinary skill in the art without creative effort shall fall within the scope of protection of the present disclosure.

An object of the present application is to provide a TIGIT polypeptide variant and a TIGIT/CTLA4 fusion protein thereof. The further objects of the present application are to provide a nucleic acid molecule encoding the polypeptide variant and the fusion protein thereof; to provide an expression vector comprising the nucleic acid molecule; to provide a host cell comprising the expression vector; to provide a method for preparing the polypeptide variant and the fusion protein thereof; to provide a pharmaceutical composition comprising the polypeptide variant and the fusion protein thereof; and to provide a medical use of the polypeptide variant and the fusion protein thereof or the pharmaceutical composition for preventing or treating immune-related diseases.

To achieve the above objects, the present application provides the following technical solutions:
A first aspect of the present application provides a TIGIT polypeptide variant, comprising a TIGIT extracellular domain or a functional fragment thereof,
wherein the variant comprises at least one amino acid mutation relative to the amino acid sequence of SEQ ID NO: 3, and the mutation position is selected from one or more of the following group: residues I42, Q56, N70, L73, G74, Y113.

In an optional embodiment, the amino acid mutation is an amino acid substitution.

In an optional embodiment, the amino acid mutation is selected from one or more of the following group: I42V, I42L, Q56N, Q56E, N70Q, N70H, L73I, L73V, G74S, Y113W, Y113T, Y113F.

In an optional embodiment, the TIGIT polypeptide variant comprises the amino acid sequence of SEQ ID NO: 21 or an amino acid sequence having at least 95%, 96%, 97%, 98%, or 99% sequence homology with the amino acid sequence of SEQ ID NO: 21.

A second aspect of the present application provides a fusion protein, comprising the TIGIT polypeptide variant.

In an optional embodiment, the fusion protein further comprises (a) a CTLA4 polypeptide or a variant thereof, and/or b) an Fc polypeptide or a variant thereof, wherein the CTLA4 polypeptide comprises an extracellular domain or a functional fragment thereof.

In an optional embodiment, the CTLA4 polypeptide variant comprises the A30Y and/or L105E amino acid mutation relative to the amino acid sequence of SEQ ID NO. 1.

In an optional embodiment, the CTLA4 polypeptide or variant thereof comprises an amino acid sequence selected from:
a)SEQ ID NO. 1;
b)SEQ ID NO. 9;
c) an amino acid sequence having at least 95%, 96%, 97%, 98%, or 99% sequence homology with SEQ ID NO: 1 or SEQ ID NO: 9.

In an optional embodiment, the Fc polypeptide is a monomer or dimer of a human IgG1, IgG2, or IgG4 Fc polypeptide.

In an optional embodiment, the Fc polypeptide comprises a hinge region, a CH2 domain, and a CH3 domain, or a CH2 domain and a CH3 domain.

In an optional embodiment, the Fc polypeptide variant comprises an amino acid mutation that improves Fc stability or half-life.

In an optional embodiment, the Fc polypeptide variant comprises one or more amino acid mutations selected from the group consisting of: M428L, N434S, M252Y, S254T, T256E, IgG4 (S228P).

In an optional embodiment, the IgG1 Fc polypeptide variant comprises the M428L, N434S, or M428L/N434S amino acid mutation.

In an optional embodiment, the IgG4 Fc polypeptide variant comprises the S228P, M428L, N434S, M428L/N434S, M428L/S228P, N434S/S228P, or M428L/N434S/S228P amino acid mutation.

In an optional embodiment, the Fc polypeptide or variant thereof comprises an amino acid sequence selected from:
a) SEQ ID NO. 7;
b) SEQ ID NO. 8;
c) an amino acid sequence having at least 95%, 96%, 97%, 98%, or 99% sequence homology with SEQ ID NO: 7 or SEQ ID NO: 8.

In an optional embodiment, the fusion protein comprises a polypeptide monomer selected from the following or a homodimer or heterodimer thereof:
a)A-L-Fc-L-B;
b)B-L-Fc-L-A;
c)A-L-B-L-Fc;
d)B-L-A-L-Fc;
wherein A is a TIGIT polypeptide variant, B is a CTLA4 polypeptide or a variant thereof, and L is absent or is a linker.

In an optional embodiment, the linker is (G4S)n or (SG4)m, wherein n or m is a positive integer selected from 1, 2, 3, 4, 5, or 6.

In an optional embodiment, the fusion protein comprises an amino acid sequence selected from SEQ ID NO: 23, SEQ ID NO: 25, SEQ ID NO: 29, or an amino acid sequence having at least 95%, 96%, 97%, 98%, or 99% sequence homology therewith.

In an optional embodiment, the fusion protein comprises the amino acid sequence of SEQ ID NO. 27, or an amino acid sequence having at least 95%, 96%, 97%, 98%, or 99% sequence homology therewith.

A second aspect of the present application provides a nucleic acid encoding the polypeptide variant or the fusion protein.

In an optional embodiment, the nucleic acid comprises a nucleic acid sequence selected from SEQ ID NO: 22, SEQ ID NO: 24, SEQ ID NO: 26, SEQ ID NO: 28, SEQ ID NO: 30.

A third aspect of the present application provides an expression vector, comprising the nucleic acid.

A fourth aspect of the present application provides a host cell, comprising the expression vector or expressing the polypeptide variant or fusion protein.

A fifth aspect of the present application provides a method for preparing the polypeptide variant or fusion protein, comprising the steps of: synthesizing the polypeptide variant or fusion protein, and/or, culturing the host cell under conditions that express the polypeptide variant or fusion protein.

A sixth aspect of the present application provides a pharmaceutical composition, comprising the polypeptide variant, the fusion protein, and optionally a pharmaceutically acceptable excipient or carrier.

A seventh aspect of the present application provides a use of the polypeptide variant, the fusion protein, or the pharmaceutical composition in the preparation of a medicament for preventing or treating an immune system disease or tumor.

In an optional embodiment, the immune system disease includes lupus erythematosus, lupus nephritis, Hashimoto's thyroiditis, diabetes (e.g., type 1 diabetes), myasthenia gravis, pemphigus, multiple sclerosis, autoimmune hemolytic anemia, idiopathic thrombocytopenia, chronic active hepatitis, ulcerative colitis, rheumatism (e.g., rheumatoid arthritis), arthritis, psoriasis, T cell lymphoma, autoimmune graft-versus-host disease (GVHD), organ transplant rejection.

An eighth aspect of the present application provides a method for preventing or treating an immune system disease or tumor, comprising administering a pharmaceutically effective amount of the polypeptide variant, the fusion protein, or the pharmaceutical composition to a subject.

In an optional embodiment, the immune system disease includes lupus erythematosus, lupus nephritis, Hashimoto's thyroiditis, diabetes (e.g., type 1 diabetes), myasthenia gravis, pemphigus, multiple sclerosis, autoimmune hemolytic anemia, idiopathic thrombocytopenia, chronic active hepatitis, ulcerative colitis, rheumatism (e.g., rheumatoid arthritis), arthritis, psoriasis, T cell lymphoma, autoimmune graft-versus-host disease (GVHD), organ transplant rejection.

In an optional embodiment, the method further comprises administering a second active molecule. The second active molecule includes, but is not limited to, an immunosuppressant, an immunomodulator, or an anti-inflammatory drug.

The objectives of the present application also include providing a TIGIT/CTLA4 fusion protein; providing a nucleic acid molecule encoding the fusion protein; providing an expression vector comprising the nucleic acid molecule; providing a host cell comprising the expression vector; providing a method for preparing the fusion protein; providing a pharmaceutical composition comprising the fusion protein; and providing a medical use of the fusion protein or the pharmaceutical composition for preventing or treating an immune system-related disease.

To achieve the above objects, the present application provides the following technical solutions:
A ninth aspect of the present application provides a fusion protein, comprising a first region and a second region, or comprising a first region, a second region, and a third region; the first region is a TIGIT polypeptide or a variant thereof, the second region is a CTLA4 polypeptide or a variant thereof, and the third region is an Fc polypeptide or a variant thereof; the TIGIT or CTLA4 comprises an extracellular domain or a functional fragment thereof.

In an optional embodiment, the TIGIT polypeptide variant comprises at least one amino acid mutation relative to the amino acid sequence of SEQ ID NO. 3, the mutation position being selected from one or more of the following group: residues I42, Q56, N70, L73, G74, Y113.

In an optional embodiment, the amino acid mutation is an amino acid substitution.

In an optional embodiment, the TIGIT polypeptide variant comprises one or more amino acid mutations selected from the following group: I42V, I42L, Q56N, Q56E, N70Q, N70H, L73I, L73V, G74S, Y113W, Y113T, Y113F.

In an optional embodiment, the TIGIT polypeptide or variant thereof comprises an amino acid sequence selected from:
a) SEQ ID NO. 3;
b) SEQ ID NO. 21;
c) an amino acid sequence having at least 95%, 96%, 97%, 98%, or 99% sequence homology with SEQ ID NO: 3 or SEQ ID NO: 21.

In an optional embodiment, the CTLA4 polypeptide variant comprises the A30Y and/or L105E amino acid mutation relative to the amino acid sequence of SEQ ID NO. 1.

In an optional embodiment, the CTLA4 polypeptide or variant thereof comprises an amino acid sequence selected from:
a) SEQ ID NO. 1;
b) SEQ ID NO. 9;
c) an amino acid sequence having at least 95%, 96%, 97%, 98%, or 99% sequence homology with SEQ ID NO: 1 or SEQ ID NO: 9.

In an optional embodiment, the Fc polypeptide is a monomer or dimer of a human IgG1, IgG2, or IgG4 Fc polypeptide.

In an optional embodiment, the Fc polypeptide comprises a hinge region, a CH2 domain, and a CH3 domain, or a CH2 domain and a CH3 domain.

In an optional embodiment, the Fc polypeptide variant comprises an amino acid mutation that improves Fc stability or half-life.

In an optional embodiment, the Fc polypeptide variant comprises one or more amino acid mutations selected from the following group: M428L, N434S, M252Y, S254T, T256E, IgG4 (S228P).

In an optional embodiment, the IgG1 Fc polypeptide variant comprises the M428L, N434S, or M428L/N434S amino acid mutation.

In an optional embodiment, the IgG4 Fc polypeptide variant comprises the S228P, M428L, N434S, M428L/N434S, M428L/S228P, N434S/S228P, or M428L/N434S/S228P amino acid mutation.

In an optional embodiment, the Fc polypeptide or variant thereof comprises an amino acid sequence selected from:
a) SEQ ID NO. 7;
b) SEQ ID NO. 8;
c) an amino acid sequence having at least 95%, 96%, 97%, 98%, or 99% sequence homology with SEQ ID NO: 7 or SEQ ID NO: 8.

In an optional embodiment, the fusion protein comprises a polypeptide monomer selected from the following or a homodimer or heterodimer thereof:
a)A-L-Fc-L-B;
b)B-L-Fc-L-A;
c)A-L-B-L-Fc;
d)B-L-A-L-Fc;
wherein A is a TIGIT polypeptide or a variant thereof, B is a CTLA4 polypeptide or a variant thereof, and L is absent or is a linker.

In an optional embodiment, the linker is (G4S)n or (SG4)m, wherein n or m is a positive integer selected from 1, 2, 3, 4, 5, or 6.

In an optional embodiment, the fusion protein comprises an amino acid sequence selected from SEQ ID NO. 13, SEQ ID NO. 15, SEQ ID NO. 17, SEQ ID NO. 19, SEQ ID NO. 23, SEQ ID NO. 25, SEQ ID NO. 29, or an amino acid sequence having at least 95%, 96%, 97%, 98%, or 99% sequence homology therewith.

In an optional embodiment, the fusion protein comprises the amino acid sequence of SEQ ID NO. 27, or an amino acid sequence having at least 95%, 96%, 97%, 98%, or 99% sequence homology therewith.

A tenth aspect of the present application provides a nucleic acid encoding the fusion protein.

An eleventh aspect of the present application provides an expression vector comprising the nucleic acid.

In an optional embodiment, the nucleic acid comprises a nucleic acid sequence selected from SEQ ID NO. 14, SEQ ID NO. 16, SEQ ID NO. 18, SEQ ID NO. 20, SEQ ID NO. 24, SEQ ID NO. 26, SEQ ID NO. 28, SEQ ID NO. 30.

A twelfth aspect of the present application provides a host cell comprising the expression vector or expressing the fusion protein.

A thirteenth aspect of the present application provides a method for preparing the fusion protein, comprising the steps of: synthesizing the fusion protein, and/or culturing the host cell under conditions that express the fusion protein.

A fourteenth aspect of the present application provides a pharmaceutical composition comprising the fusion protein and optionally a pharmaceutically acceptable excipient or carrier.

A fifteenth aspect of the present application provides use of the fusion protein or the pharmaceutical composition in the preparation of a medicament for preventing or treating an immune system disease or tumor.

In an optional embodiment, the immune system disease includes lupus erythematosus, lupus nephritis, Hashimoto's thyroiditis, diabetes (e.g., type 1 diabetes), myasthenia gravis, pemphigus, multiple sclerosis, autoimmune hemolytic anemia, idiopathic thrombocytopenia, chronic active hepatitis, ulcerative colitis, rheumatism (e.g., rheumatoid arthritis), arthritis, psoriasis, T cell lymphoma, autoimmune graft-versus-host disease (GVHD), organ transplant rejection.

A sixteenth aspect of the present application provides a method for preventing or treating an immune system disease or tumor, comprising administering a pharmaceutically effective amount of the fusion protein or the pharmaceutical composition to a subject.

In an optional embodiment, the immune system disease includes lupus erythematosus, lupus nephritis, Hashimoto's thyroiditis, diabetes (e.g., type 1 diabetes), myasthenia gravis, pemphigus, multiple sclerosis, autoimmune hemolytic anemia, idiopathic thrombocytopenia, chronic active hepatitis, ulcerative colitis, rheumatism (e.g., rheumatoid arthritis), arthritis, psoriasis, T cell lymphoma, autoimmune graft-versus-host disease (GVHD), organ transplant rejection.

In an optional embodiment, the method further comprises administering a second active molecule. The second active molecule includes, but is not limited to, an immunosuppressant, an immunomodulator, or an anti-inflammatory drug.

In an optional embodiment, the aforementioned immune system disease or tumor is associated with the CTLA-4 and/or TIGIT signaling pathway.

In an optional embodiment, the aforementioned immune system disease is an autoimmune disease.

The amino acid sequences in the present application are arranged from the N-terminus to the C-terminus.

It should be understood that, within the scope of the present application, the above technical features of the present application and the technical features specifically described below (e.g., in the Examples) can be combined with each other to constitute new or preferred technical solutions. Due to space limitations, they are not listed one by one here.

### Definitions

Unless otherwise defined, all technical and scientific terms used herein have the same meaning as commonly understood by one of ordinary skill in the art to which this application belongs.

### TIGIT

The term "TIGIT" refers to naturally occurring full-length TIGIT or a fragment thereof. Full-length TIGIT is a cellular transmembrane protein, consisting of an extracellular immunoglobulin variable (IgV) domain (i.e., the extracellular domain), a type 1 transmembrane domain, and an intracellular domain comprising a classical immunoreceptor tyrosine-based inhibitory motif (ITIM) and an immunoglobulin tyrosine tail (ITT) motif. The "TIGIT extracellular domain or functional fragment thereof" refers to a portion capable of binding to target antigens CD155, CD112, and/or CD113.In a specific embodiment, the TIGIT extracellular domain refers to Met22-Pro141 of NCBI Gene Accession No. NP_776160.2.

### CTLA4

The term "CTLA4" refers to naturally occurring full-length CTLA4 or a fragment thereof. Full-length CTLA4 is a cell surface protein, comprising an N-terminal extracellular domain, a transmembrane domain, and a C-terminal cytoplasmic domain. The "CTLA4 extracellular domain or functional fragment thereof" refers to a portion capable of binding to target antigens CD80 and/or CD86.In a specific embodiment, the CTLA4 extracellular domain refers to Ala37-Phe162 of Gene Accession No. Q6GR94.

### Variants

The term "variant" refers to a peptide comprising at least one amino acid substitution, deletion, or insertion relative to a wild-type or naturally occurring peptide. For example, conservative variants well known to those skilled in the art.

### Sequence Identity

Methods for determining sequence identity known to those of ordinary skill in the art include, but are not limited to: Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M. and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H. and Lipman, D., SIAM J. Applied Math., 48:1073 (1988). Preferred methods for determining identity are designed to give the largest match between the sequences tested. Methods for determining identity are compiled in publicly available computer programs. Preferred computer program methods for determining identity between two sequences include, but are not limited to: the GCG program package (Devereux, J. et al., 1984), BLASTP, BLASTN, and FASTA (Altschul, S.F. et al., 1990). The BLASTX program is publicly available from NCBI and other sources (BLAST Manual, Altschul, S. et al., NCBI NLM NIH Bethesda, Md. 20894; Altschul, S. et al., 1990). The well-known Smith Waterman algorithm can also be used to determine identity.

### Fusion Protein

The term "fusion protein" refers to a new polypeptide sequence obtained by fusing a plurality of identical or different polypeptide sequences. The term "fusion" refers to connection directly via a peptide bond or via one or more linkers (peptide linkers). The term "linker (peptide linker)" refers to a short peptide capable of connecting two polypeptide sequences, generally having a length of 1-30 amino acids. Preferably, the peptide linker is a flexible peptide linker. Examples of suitable linkers include single glycine (Gly) or serine (Ser) residues, and the identity and sequence of amino acid residues in the linker can vary depending on the type of secondary structural element to be achieved in the linker.

### A Plurality

The term "a plurality" refers to at least two.

### Encoding Nucleic Acids and Expression Vectors

The present application also provides a nucleic acid molecule encoding the above-mentioned fusion protein. The nucleic acid of the present application may be in the form of DNA or RNA. The DNA form includes cDNA, genomic DNA, or synthetic DNA. The DNA may be single-stranded or doublestranded. The DNA may be a coding strand or a non-coding strand. The sequence of the DNA molecule of the fusion protein of the present application can be obtained using conventional techniques, for example, by PCR amplification. Furthermore, the relevant sequences can also be synthesized by artificial synthesis methods.

The present application also relates to a vector comprising the appropriate DNA sequence described above and an appropriate promoter or control sequence. These vectors can be used to transform an appropriate host cell to enable it to express the protein. The vector is a conventional expression vector in the art, referring to an expression vector comprising appropriate regulatory sequences, such as promoter sequences, terminator sequences, polyadenylation sequences, enhancer sequences, marker genes and/or sequences, and other appropriate sequences. The expression vector may be a virus or a plasmid, such as an appropriate phage or phagemid. For more technical details, see, for example, Sambrook et al., Molecular Cloning: A Laboratory Manual, Second Edition, Cold Spring Harbor Laboratory Press, 1989. Many known techniques and protocols for nucleic acid manipulation can be found in Current Protocols in Molecular Biology, Second Edition, edited by Ausubel et al. The expression vector of the present application is preferably pcDNA3.4, pDR1, pcDNA3.1(+), pcDNA3.1/ZEO(+), pDHFR, pcDNA4, pDHFF, pGM-CSF, or pCHO 1.0.

In the present application, the term "host cell" refers to various conventional host cells in the art, as long as they allow the vector to stably replicate itself and the polynucleotide molecule carried therein can be effectively expressed. The host cell includes prokaryotic expression cells and eukaryotic expression cells, and the host cell preferably includes: COS, CHO, NS0, sf9, sf21, DH5α, BL21(DE3), TG1, BL21(DE3), 293F, or 293E cells.

### Pharmaceutical Composition

The term "pharmaceutical composition" refers to a single or compound pharmaceutical preparation composed of the polypeptide or variant thereof, or fusion protein of the present application together with a pharmaceutically acceptable excipient or carrier. The excipient or carrier includes excipients or carriers well known to those skilled in the art. Suitable carriers and excipients include, but are not limited to, human serum albumin, saline, buffers, glucose, water, glycerol, ethanol, glycine, sorbic acid, potassium sorbate, and combinations thereof.

The pharmaceutical composition of the present application can be in various dosage forms, including but not limited to liquid solutions or suspensions, injections, injectables, and liposomal formulations. The preferred dosage form depends on the mode of administration and the purpose of treatment. The pharmaceutical composition of the present application can be administered to a subject using conventional modes of administration, including but not limited to intravenous administration, intraperitoneal administration, intramuscular administration, and subcutaneous administration.

Through multiple rounds of protein engineering and screening, the present application has obtained a bispecific fusion protein CTLA4-TIGIT that can competitively inhibit the activation of the CD28-CD80/86 and CD226-CD155 pathways, exerting a synergistic inhibitory effect on the activation of CD4⁺ and CD8⁺ T cells that surpasses that of CTLA4 or TIGIT fusion proteins used alone, and will exert more effective and broader therapeutic effects in related autoimmune diseases.

Compared with the related art, the beneficial technical effects of the present application include: The present application
1. Through extensive research and analysis, the present application has identified key amino acid sites that affect the stability of the "LOCK-and-KEY" interface of the TIGIT/CD155 interaction. On this basis, TIGIT polypeptide variants with significantly enhanced affinity and slower dissociation kinetics were obtained through multiple rounds of screening, thereby achieving unexpected technical advantages. The TIGIT polypeptide variants have superior biological activities, including a stronger ability to block the CD226/CD155 signaling pathway and the ability to exert stable immunomodulatory activity at lower doses. When used in the clinical treatment of immune system diseases, benefits can be obtained in multiple aspects, including efficacy, safety, stability, and durability.
2. The present application provides a new dual-target combination scheme, constructing a novel fusion protein by combining the TIGIT extracellular domain protein and the CTLA4 extracellular domain protein for the first time. The TIGIT/CTLA4 fusion protein of the present application can better inhibit T cell activation/proliferation and inflammatory cytokine secretion by simultaneously blocking the CD226/CD155 and CD80/CD86-CD28 signaling pathways. This addresses clinical problems that are unattainable with monotherapy or combination therapy. As demonstrated by the exemplary TIGIT/CTLA4 fusion protein, it possesses signaling pathway inhibition ability and T cell inhibitory activity superior to each single-target fusion protein, and its T cell inhibitory activity is superior to the combination of TIGIT fusion protein and CTLA4 fusion protein, exhibiting unexpected synergistic or additive activity.

In cell experiments or animal models of immune-related diseases, such as arthritis models, it has demonstrated in vivo or in vitro effects superior to existing drug molecules, such as Abatacept and Belatacept.

The present application is further described below in conjunction with specific examples. It should be understood that these examples are only for illustrating the present application and are not intended to limit the scope of the present application. Experimental methods for which detailed conditions are not specified in the following examples are generally performed under conventional conditions, such as those described in Sambrook et al., Molecular Cloning: A Laboratory Manual (New York: Cold Spring Harbor Laboratory Press, 1989), or under conditions recommended by the manufacturer.

### Example 1 Preparation of Fusion Proteins

The extracellular domain of CTLA4 (Ala37-Phe162, NCBI Gene ID: Q6GR94; SEQ ID NO. 1), the extracellular domain of TIGIT (Met22-Pro141, NCBI Gene ID: NP_776160.2; SEQ ID NO. 3), the extracellular domain of CD28 (Asn19-Pro152, UniProtKB ID: P10747; SEQ ID NO. 5), and the extracellular domain of CD226 (Glu19-Asn247, UniProtKB ID: Q15762.2; SEQ ID NO. 6) were respectively linked to the constant region Fc of human IgG1 (SEQ ID NO. 7) or to the constant region Fc of human IgG4 (comprising the S228P mutation, SEQ ID NO. 8) via gene synthesis and molecular cloning methods, and cloned into the pcDNA3.4 vector. HEK-293F cells were transfected and cultured in a cell shaker incubator at 37°C, 8% CO₂ for protein expression. After 5 days, the cell supernatant was collected by centrifugation, and the target protein was purified using a Protein A affinity chromatography column to obtain each fusion protein. Fusion proteins linked to the constant region Fc of human IgG1 were named with the suffix -Fc, and fusion proteins linked to the constant region Fc of human IgG4 were named with the suffix -FcG4. For example, the IgG1 and IgG4 subtype fusion proteins of CTLA4 were named CTLA4-Fc and CTLA4-FcG4, respectively.

The C-termini of the extracellular domain of CD86 (Leu20-Asp238, UniProtKB ID: P42081; SEQ ID NO. 11) and the extracellular domain of CD155 (Gly27-Asn343, UniProtKB ID: P15151; SEQ ID NO. 12) were respectively linked to 6×His and cloned into the pcDNA3.4 vector. HEK-293F cells were transfected, and CD86-His and CD155-His proteins were obtained through expression and purification, respectively.

### Example 2 Affinity Determination of CTLA4 and TIGIT Fusion Proteins

In this example, the affinity of each fusion protein for its corresponding ligand was determined using an enzyme-linked immunosorbent assay (ELISA). Specifically, CD86-His and CD155-His proteins were diluted to 2 and 5 µg/mL, respectively, with ELISA coating buffer, and added to an ELISA plate at 100 µL/well, which was then placed in a humidified box at 4°C for 16 h for coating. The ELISA plate was washed three times with PBST to remove unbound antigen, and patted dry on absorbent paper to remove excess liquid. Then, 2% BSA prepared in PBS was added at 200 µL/well for blocking at room temperature for 2 h. After washing three times with PBST to remove excess blocking solution and patting dry on absorbent paper, each test protein was diluted with 1% BSA prepared in PBST. For CTLA4- and CD28-related fusion proteins, the highest working concentration was 3600 nM, and 11 gradients were prepared by 5-fold serial dilution, which were then added to the ELISA plate coated with CD86-His. For TIGIT- and CD226-related fusion proteins, the highest working concentration was 5000 nM, and 11 concentrations were prepared by 2.5-fold serial dilution, which were then added to the ELISA plate coated with CD155-His, at 100 µL/well, and incubated at room temperature for 1 h. Each sample was tested in duplicate. After washing off unbound or non-specifically bound samples, an HRP-labeled anti-human Fc secondary antibody was diluted 1:5000 with antibody diluent according to the antibody manufacturer's instructions, added to each of the above ELISA plates at 100 µL/well, and incubated at room temperature for 1 h. After washing three times with PBST and patting dry on absorbent paper to remove excess liquid, TMB chromogenic solution (purchased from KPL, catalog number 5120-0075) was added at 100 µL/well. After color development to an appropriate intensity, 2M H₂SO₄ was added at 50 µL/well to stop the reaction. The absorbance was measured at a wavelength of 450 nm using a multifunctional microplate reader. Data were analyzed using GraphPad Prism 10 with the "log(agonist) vs. response -- Variable slope (four parameters)" parameter setting.

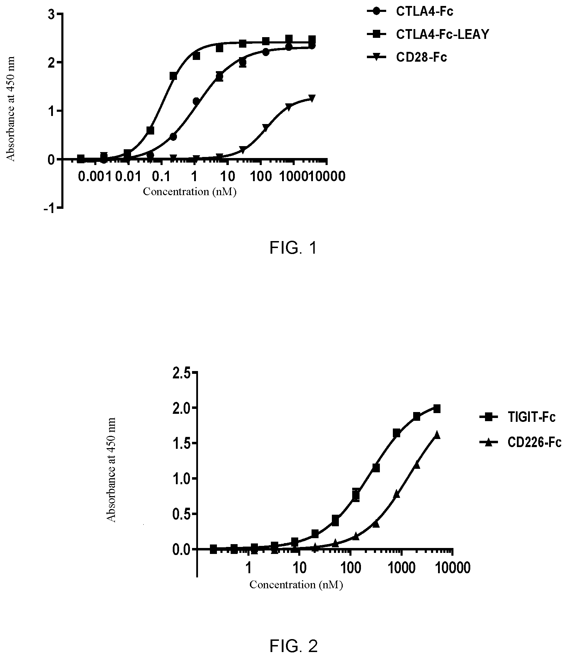

The results of the affinity determination of CTLA4-LEAY-Fc, CD28-Fc, and CTLA4-Fc for CD86-His are shown in FIG. 1. As shown in FIG. 1, the affinity of CTLA4-LEAY-Fc for CD86-His was stronger than that of CTLA4-Fc and CD28-Fc, wherein the EC₅₀ values of CTLA4-LEAY-Fc, CTLA4-Fc, and CD28-Fc were 0.11 nM, 1.24 nM, and 154.35 nM, respectively.

The results of the affinity determination of TIGIT-Fc and CD226-Fc for CD155-His are shown in FIG. 2. As shown in FIG. 2, the affinity of TIGIT-Fc for CD155-His was stronger than that of CD226-Fc, wherein the EC₅₀ values of TIGIT-Fc and CD226-Fc were 244 nM and 1465.5 nM, respectively.

### Example 3 Preparation of Bispecific Fusion Proteins

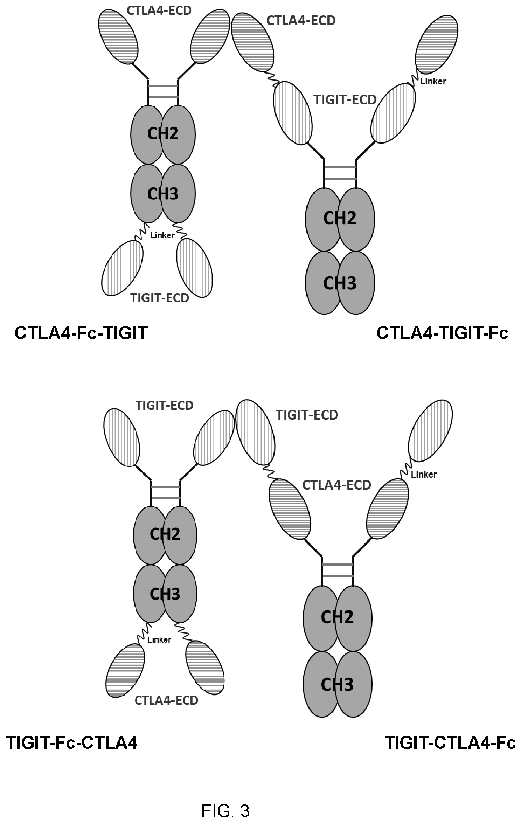

Using gene synthesis and molecular cloning techniques, the extracellular domains of CTLA4 and TIGIT, as well as the human IgG1 constant region Fc, were constructed into multiple bispecific molecules via a (G4S)₃Linker according to different structural formats. After transfecting HEK-293F cells, the bispecific fusion proteins were obtained by referring to the protein expression and purification method of Example 1, and were respectively named CTLA4-Fc-TIGIT (SEQ ID NO. 13), CTLA4-TIGIT-Fc (SEQ ID NO. 15), TIGIT-CTLA4-Fc (SEQ ID NO. 17), and TIGIT-Fc-CTLA4 (SEQ ID NO. 19). Schematic diagrams of the structures of the bispecific fusion proteins CTLA4-Fc-TIGIT, CTLA4-TIGIT-Fc, TIGIT-CTLA4-Fc, and TIGIT-Fc-CTLA4 are shown in FIG. 3.

The expression levels of each of the above bispecific fusion proteins expressed in HEK-293F and the purity of the samples after one-step Protein A (PrtA) purification as determined by UPLC are respectively summarized in Table 1. It can be seen that the transient expression level and the sample purity after one-step PrtA purification of the bispecific fusion protein TIGIT-CTLA4-Fc were relatively superior.

**Table 1 Expression Parameters of Bispecific Fusion Proteins**

| **Sample** | **Expression Level (mg/L)** | **UPLC after One-Step PrtA (%)** |
|---|---|---|
| CTLA4-Fc-TIGIT | 18 | 62 |
| CTLA4-TIGIT-Fc | 5 | 32 |
| TIGIT-CTLA4-Fc | 28 | 77 |
| TIGIT-Fc-CTLA4 | 14 | 79 |

### Example 4 Affinity Determination of Bispecific Fusion Proteins

Referring to the ELISA detection method of Example 2, the affinities of CTLA4-Fc-TIGIT, CTLA4-TIGIT-Fc, TIGIT-CTLA4-Fc, and TIGIT-Fc-CTLA4 for CD86-His and CD155-His were respectively determined.

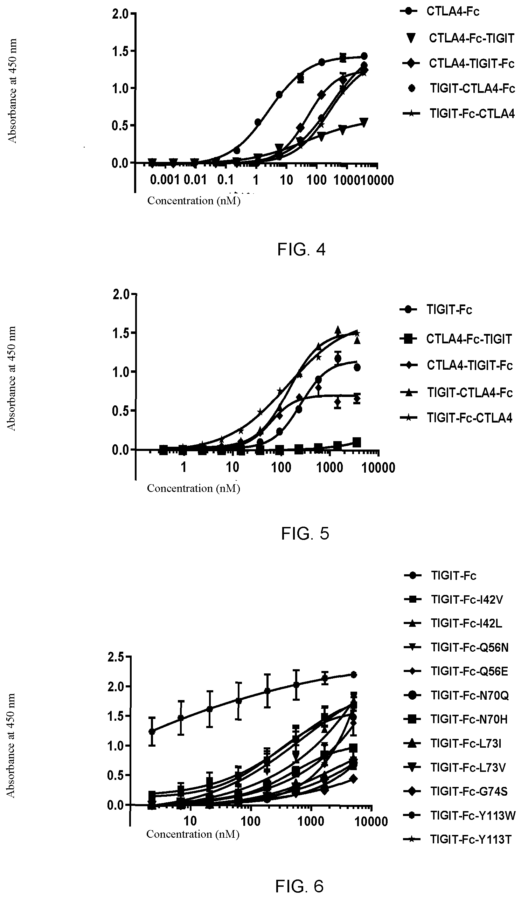

As shown in FIG. 4, among the bispecific fusion proteins, CTLA4-TIGIT-Fc exhibited the best affinity for CD86-His, with an EC₅₀ of 49.78 nM; followed by TIGIT-CTLA4-Fc and TIGIT-Fc-CTLA4, whose EC₅₀ values were 308.4 nM and 260.6 nM, respectively; CTLA4-Fc-TIGIT showed the weakest affinity for CD86-His, but the affinities of all bispecific fusion proteins for CD86-His were significantly weaker than that of the control single molecule CTLA4-Fc, for which the EC₅₀ was 2.64 nM. This may be related to the steric hindrance caused by the position of CTLA4 in the bispecific fusion proteins and the bridging effect generated by the detection secondary antibody.

As shown in FIG. 5, among the bispecific fusion proteins, TIGIT-CTLA4-Fc and TIGIT-Fc-CTLA4 exhibited the best affinities for CD155-His, with EC₅₀ values of 140.85 nM and 134 nM, respectively, which were superior to that of the control single molecule TIGIT-Fc, whose EC₅₀ was 249.4 nM. CTLA4-Fc-TIGIT showed the weakest affinity for CD155-His.

### Example 5 Engineering of TIGIT Fusion Proteins

Based on the three-dimensional structural analysis results of the TIGIT/CD155 complex by Katharina F. Stengel (Stengel, et al. Proc. Natl. Acad. Sci. USA 2012, 109, 5399), and utilizing the AI (Artificial Intelligence) algorithm of Wecomput's Protein Virtual Affinity Maturation platform, engineering was performed on potential amino acid sites affecting the stability of the "LOCK-and-KEY" interaction interface of TIGIT/CD155.

Through comprehensive analysis, in the first round, the following amino acid sites were selected for site-directed mutagenesis based on TIGIT-Fc: I42V, I42L, Q56N, Q56E, N70Q, N70H, L73I, L73V, G74S, Y113W, and Y113T.The TIGIT-Fc mutant pcDNA3.4 expression vectors obtained through the above site-directed mutagenesis cloning were transfected into HEK-293F cells, expressed, and purified to obtain each TIGIT-Fc mutant protein. The affinity of each mutant for CD155 was detected according to the ELISA detection method of Example 2.

As shown in the results of FIG. 6, among the TIGIT-Fc mutant proteins, TIGIT-Fc-Y113W exhibited the highest affinity for CD155-His, i.e., mutating the 113th Tyr (Y) to Trp (W) in the TIGIT extracellular domain (designated as TIGIT extracellular domain-Y113W, SEQ ID NO. 21) could significantly improve the affinity of TIGIT for CD155, which was markedly superior to TIGIT-Fc.

Based on the above first-round screening results, the 113th Y in the "KEY" domain of the TIGIT protein was selected for a second round of mutagenesis according to the principle of "similarity and proximity, maximum difference". The specific mutation information is as follows: Y113W, Y113H, Y113E, Y113K, Y113A, Y113F. The above site-directed mutagenesis clones were constructed into the pcDNA3.4 vector, transfected into HEK-293F cells, expressed, and purified to obtain each mutant protein. The affinity of each mutant for CD155 was detected according to the ELISA method of Example 2.

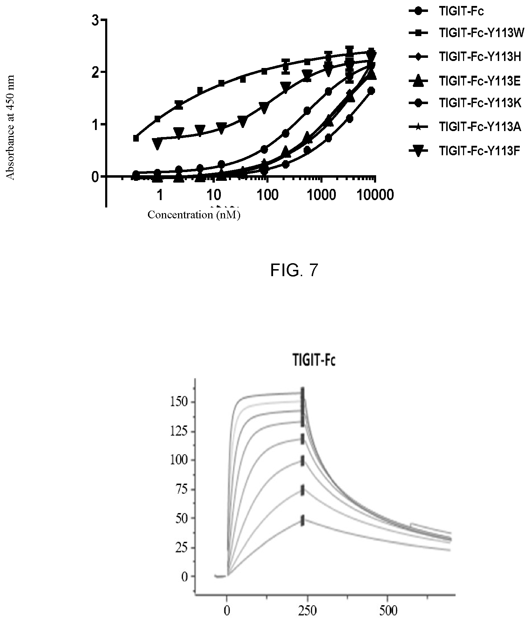

As shown in the results of FIG. 7, TIGIT-Fc-Y113W exhibited the optimal affinity for CD155, with its EC₅₀ reaching less than 1 nM; followed by TIGIT-Fc-Y113F, whose EC₅₀ was 128.5 nM. The EC₅₀ and the top and bottom plateau values for each sample were obtained through four-parameter curve fitting analysis using GraphPad Prism, as shown in Table 2.

**Table 2 EC₅₀ of Binding of Each TIGIT Mutant Protein to CD155**

| **Sample** | **Bottom** | **Top** | **EC₅₀(nM)** |
|---|---|---|---|
| TIGIT-Fc | 0.06 | 2.36 | 513.10 |
| TIGIT-Fc-Y113W | -365.50 | 2.59 | < 1 |
| TIGIT-Fc-Y113H | -0.02 | 2.65 | 1992.00 |
| TIGIT-Fc-Y113E | -0.05 | 2.93 | 2767.00 |
| TIGIT-Fc-Y113F | 0.70 | 2.27 | 128.50 |
| TIGIT-Fc-Y113K | -0.03 | 3.38 | 9186.00 |
| TIGIT-Fc-Y113A | -0.05 | 6.53 | 31970.00 |

### Example 6 Binding Kinetics of TIGIT Fusion Proteins

Based on the above experimental results, TIGIT-Fc-Y113W was selected for further analysis of its binding kinetics to the CD155 protein using a Biacore 8K. The specific experimental procedure is as follows:
First, the CM5 chip was used to capture the CD155-Fc protein, with relevant operating parameters as follows: the concentration of CD155-Fc was 12 µg/mL, the dilution buffer used was 10 mM sodium acetate at pH 4.0, the contact time was 300 s, and the flow rate was 10 µL/min; subsequently, the test proteins TIGIT-Fc-Y113W and TIGIT-Fc were diluted using HBS-EP+ pH 7.4 + 150 mM NaCl buffer, with the highest concentration set at 50 nM, serially diluted 2-fold to 0.1953125, and a 0 concentration point was set; the highest concentration for the test protein CD226-Fc was set at 8000 nM, serially diluted 2-fold to 31.25 nM, and a 0 concentration point was set; 0.2 M NaOH solution was used as the regeneration buffer, and injection was performed on the Biacore 8K with the following parameters: association time of 240 s, dissociation time of 450 s, flow rate of 30 µL/min, and regeneration contact time of 40 s at a flow rate of 30 µL/min. Finally, the collected data were analyzed using the Biacore 8K Evaluation Software.

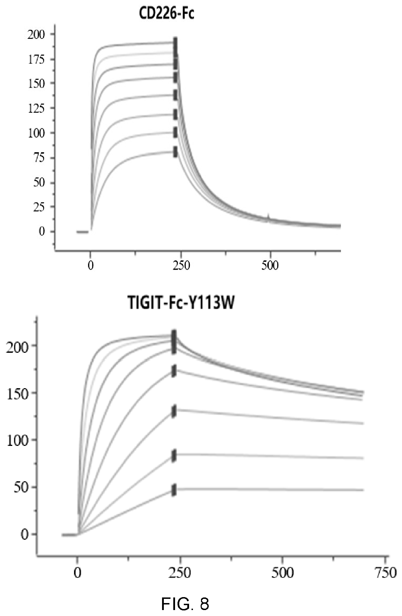

The binding and dissociation curves of each protein with CD155 are detailed in FIG. 8, and the binding kinetic parameters are summarized in Table 3. As shown in Table 3, through analysis of the hydrophobic/hydrophilic interactions of the key amino acids forming the "key-lock" between TIGIT and CD155, a mutant TIGIT-Fc-Y113W with stronger affinity for CD155 was successfully screened and obtained. Compared to the wild-type TIGIT-Fc, the affinity (KD) was improved by approximately 2.5-fold, but the dissociation rate of TIGIT-Fc-Y113W was slower, being about 7.5-fold better than TIGIT-Fc and about 135-fold better than CD226-Fc, suggesting that TIGIT-Fc-Y113W may possess superior biological activity.

**Table 3 Binding Kinetic Parameters of Fusion Proteins to CD155**

| **Sample** | **ka (1/Ms)** | **kd (1/s)** | **KD (M)** |
|---|---|---|---|
| TIGIT-Fc | 5.01E+06 | 8.71E-03 | 1.74E-09 |
| TIGIT-Fc -Y113W | 1.62E+06 | 1.15E-03 | 7.10E-10 |
| CD226-Fc | 2.15E+05 | 2.09E-02 | 9.75E-08 |

### Example 7 Blocking Effect of TIGIT Protein Mutants on the CD226/CD155 Signaling Pathway TIGIT

CHO cells overexpressing CD155 and TCR Activator (purchased from Nanjing Cobioer, catalog number: CBP74073) and Jurkat cells overexpressing the NFAT-Luc fluorescent reporter system (purchased from Genomeditech, catalog number: GM-C01459) were co-cultured. At the experimental endpoint, the Luciferase expression level resulting from activation of the CD226/CD155 downstream NFAT pathway was determined by adding a Luciferin substrate and detecting the fluorescent signal. The specific experimental procedure is as follows:
CD155/TCR Activator/CHO cells in the logarithmic growth phase were collected, centrifuged, washed once with DPBS, and then resuspended in F12 medium containing 1% FBS to an appropriate concentration. The cells were plated at 100 µL per well and cultured overnight at 37°C. The next day, the 96-well cell culture plate seeded with CD155/TCR Activator/CHO cells was taken out, the culture medium was discarded, and the test fusion protein prepared in F12 medium containing 1% FBS was added at a concentration of 9000 nM, 50 µL/well, and incubated for 30 minutes at room temperature. Finally, NFAT-Luc Jurkat cells in the logarithmic growth phase were collected, centrifuged, washed once with DPBS, and then resuspended in F12 medium containing 1% FBS to an appropriate concentration (the ratio of CD155/TCR Activator/CHO cells to NFAT-Luc Jurkat cells was 1:8). The cells were added to the above 96-well plate at 50 µL/well, gently mixed, and placed in a 37°C incubator for incubation. After 6 hours, Bio-Glu reagent (purchased from Promega, catalog number G7940) was added to the wells at 100 µL/well, and the fluorescent signal was detected using a microplate reader for data analysis.

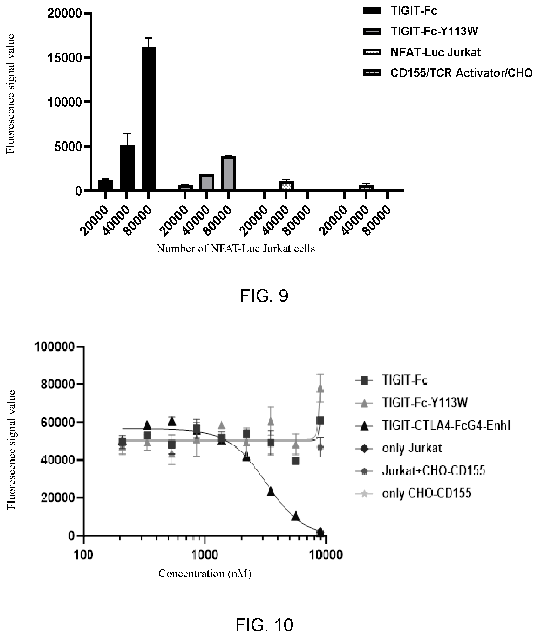

As shown in FIG. 9, under different numbers of effector cells (NFAT-Luc Jurkat), the activity of TIGIT-Fc-Y113W in blocking CD226/CD155 downstream signal transduction was significantly superior to that of TIGIT-Fc.

### Example 8 Preparation of Novel Bispecific Fusion Proteins

Following the method of Example 3, Ala at position 30 and Leu at position 105 on CTLA4 of CTLA4-Fc-TIGIT, CTLA4-TIGIT-Fc, TIGIT-CTLA4-Fc, and TIGIT-Fc-CTLA4 were mutated to Tyr and Glu, respectively, Tyr at position 113 on TIGIT was mutated to Trp, and M428L and N434S mutations were simultaneously introduced into the Fc region to achieve an enhanced in vivo half-life of the samples. The resulting novel bispecific fusion proteins were designated as CTLA4-Fc-TIGIT-Enhl, CTLA4-TIGIT-Fc-Enhl, TIGIT-CTLA4-Fc-Enhl, and TIGIT-Fc-CTLA4-Enhl, respectively. Furthermore, considering that the mechanism of action of this target may require avoiding the biological functional effects of Fc, mutations were simultaneously introduced to replace the Fc region of the aforementioned novel bispecific fusion proteins with human IgG4(S228P) Fc containing M428L and N434S, designated as CTLA4-FcG4-TIGIT-Enhl (SEQ ID NO. 23), CTLA4-TIGIT-FcG4-Enhl (SEQ ID NO. 25), TIGIT-CTLA4-FcG4-Enhl (SEQ ID NO. 27), and TIGIT-FcG4-CTLA4-Enhl (SEQ ID NO. 29), respectively. The expression data statistics for the transient transfection of the above novel bispecific fusion proteins in HEK-293F are shown in Table 4.As shown in Table 4, TIGIT-CTLA4-Fc-Enhl had the highest transient expression level and relatively high purity after one-step Protein A purification. The protein expression results for the Fc region replaced with IgG4(S228P) Fc were similar, with TIGIT-CTLA4-FcG4-Enhl also showing a high expression level and high protein purity after one-step Protein A purification.

**Table 4 Expression Parameters of Novel Bispecific Fusion Proteins**

| **Sample** | **Expression Level (mg/L)** | **UPLC after One-Step PrtA (%)** |
|---|---|---|
| CTLA4-Fc-TIGIT-Enhl | 2 | 98 |
| TIGIT-CTLA4-Fc-Enhl | 68 | 94 |
| TIGIT-Fc-CTLA4-Enhl | 10 | 71 |

### Example 9 Blocking Effect of Bispecific Fusion Proteins on Downstream Signaling Pathways

Based on the expression results of the bispecific fusion proteins described above, the bispecific fusion protein TIGIT-CTLA4-FcG4-Enhl was selected for further study. The cellular functional activity of its TIGIT moiety can be assessed by co-culturing CD155/TCR Activator/CHO cells and NFAT-Luc Jurkat cells, with the specific experimental method referring to Example 7. As shown in FIG. 10, the bispecific fusion protein TIGIT-CTLA4-FcG4-Enhl exhibited significantly superior inhibitory activity on CD226/CD155 downstream signal transduction compared to the single fusion proteins TIGIT-Fc and TIGIT-Fc-Y113W. The IC₅₀ of TIGIT-CTLA4-FcG4-Enhl, determined by four-parameter curve fitting analysis, was 3191 nM.

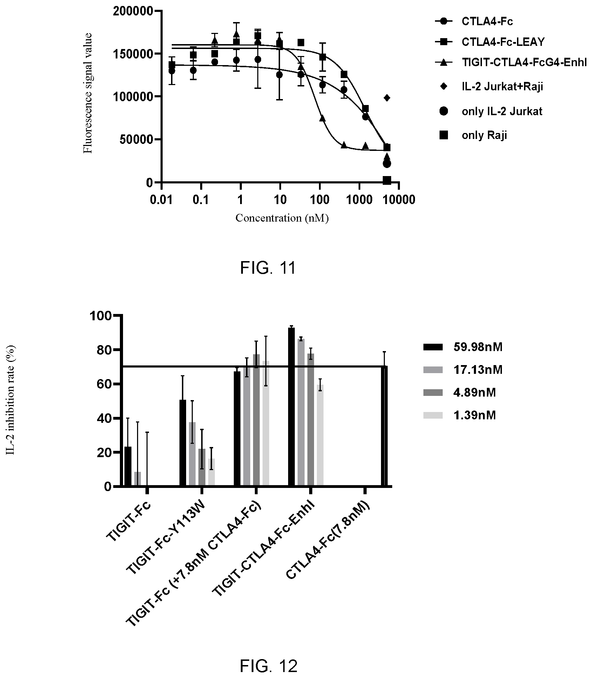

The cellular functional activity of the CTLA4 moiety of TIGIT-CTLA4-FcG4-Enhl can be assessed by co-culturing Raji cells (purchased from Nanjing Kebai, catalog number: CBP60272) and IL-2-Luc Jurkat cells. Raji cells highly express CD80 and CD86 proteins on their surface, and IL-2-Luc Jurkat cells overexpress CD28 protein on their surface. The CD80/CD86-CD28 interaction is an important co-stimulatory signal during T cell activation. In a co-culture system, the activation level of the CD80/CD86-CD28 signaling pathway can be determined by measuring the expression level of the Luciferase gene activated by the downstream IL-2 promoter. The specific experimental procedure is as follows:
First, collect Raji cells in the logarithmic growth phase, wash them once with DPBS after centrifugation, and then resuspend the cells in serum-free RPMI 1640 medium. Plate the cells in a 96-well cell culture plate at 6,000 cells per well. Subsequently, add 40 µL of anti-CD3 antibody (purchased from Invitrogen, catalog number: 14-0037-82) at a working concentration of 53.33 ng/mL. Prepare 11 concentration gradients of the test samples by performing 3.5-fold serial dilutions starting from a highest working concentration of 5000 nM, add them to the aforementioned cell culture plate, and incubate at room temperature for 30 min. Collect IL-2-Luc Jurkat cells in the logarithmic growth phase, wash them once with DPBS after centrifugation, and then resuspend the cells in serum-free RPMI 1640 medium. Add the cells to the aforementioned cell culture plate at 120,000 cells per well. Gently shake to mix, and then place in a 37°C incubator for incubation, with a final total volume of 120 µL/well. After 20 h of incubation, add 100 µL of Bio-Glo reagent (purchased from Promega, catalog number G7940) to detect luminescence. The experimental results are shown in FIG. 11. The bispecific fusion protein TIGIT-CTLA4-FcG4-Enhl exhibited significantly superior inhibitory activity on CD80/CD86-CD28 downstream signal transduction compared to the single fusion proteins CTLA4-Fc and CTLA4-Fc-LEAY. Through four-parameter curve fitting analysis, the IC₅₀ values for TIGIT-CTLA4-FcG4-Enhl, CTLA4-Fc, and CTLA4-Fc-LEAY were determined to be 74.47 nM, 1976 nM, and 1409 nM, respectively.

### Example 10 Inhibitory Function of Bispecific Fusion Proteins in Mature DC Cell-Mediated T Cell Activation

During the process of mature DC-induced T cell activation, the activation of co-stimulatory signaling pathways is crucial for T cell activation. The TIGIT-CTLA4-FcG4-Enhl bispecific fusion protein can competitively bind to CD155 and CD80/CD86, respectively, thereby blocking the activation of these two co-stimulatory signaling pathways. In this example, the biological function of the fusion protein is detected by measuring the concentration of IL-2 secreted into the cell supernatant during DC-induced T cell activation. The specific experimental procedure is as follows:
Prepare a 96-well cell culture plate coated with 5 µg/mL anti-human CD3 antibody (purchased from Invitrogen, catalog number 14-0037-82), seal with parafilm, and coat overnight at 4°C; before adding the isolated T cells, discard the liquid in the wells and wash twice with 200 µL of sterile PBS. According to the manufacturer's instructions, isolate CD4+ T cells from fresh PBMCs using a negative selection kit (purchased from STEMCELL, catalog number:
17952); isolate CD8+ T cells from fresh PBMCs using a positive selection kit (purchased from STEMCELL, catalog number: 17853); isolate CD14+ cells from fresh PBMCs using CD14 microbeads (purchased from Miltenyi Biotec, catalog number 130-050-201); induce the CD14+ cells to differentiate into DCs by culturing with 50 ng/mL human IL-4 (purchased from R&D, catalog number 204-IL-020/CF) and 50 ng/mL rhGM-CSF (purchased from R&D, catalog number 7954-GM-020/CF) for 7 days, changing the medium every three days, and on day 8, induce with 50 ng/mL rhGM-CSF + 50 ng/mL IL-4 + 100 ng/mL LPS (purchased from Merck, catalog number L2630-25MG) for 1-2 days to obtain mature DCs. Resuspend the obtained mature DCs in RPMI 1640 complete medium to 3.33 × 10⁵ cells/mL, add 50 µL per well to the 96-well plate coated with CD3 antibody, add the test protein dilutions, incubate at room temperature for 30 min, then resuspend the obtained CD4+ T cells or CD8+ T cells in RPMI 1640 complete medium to 1 × 10⁶ cells/mL, add 50 µL per well to the aforementioned 96-well plate, incubate for 1 h, gently mix, and culture in a 37°C incubator. After 24 h, collect the cell culture supernatant for IL-2 detection. The IL-2 detection experimental procedure is as follows: Dilute the human IL-2 antibody (purchased from Invitrogen, catalog number 14-7029-81) to 1 µg/mL with ELISA coating buffer, coat the ELISA plate at 100 µL/well, place in a humidified box, and coat at 4°C for 16 h; wash the ELISA plate three times with PBST to remove unbound antigen, pat the ELISA plate dry on absorbent paper to remove excess liquid, then block with 2% BSA prepared in PBS at 200 µL/well at room temperature for 2 h; wash once with PBST to remove excess blocking solution, pat the ELISA plate dry to remove excess liquid, add the cell supernatant, and simultaneously dilute the IL-2 protein standard (purchased from R&D, catalog number 202-IL-050/CF) starting from a concentration of 500 ng/mL in a 3-fold serial dilution for 15 concentration gradients, at 100 µL/well, incubate at room temperature for 1.5 h; wash the ELISA plate three times with PBST and pat dry, dilute the biotinylated IL-2 antibody (purchased from Invitrogen, catalog number M600B) to 1 µg/mL with antibody diluent, add to the ELISA plate at 100 µL/well, incubate at room temperature for 1 h; wash the ELISA plate three times with PBST and pat dry, dilute the HRP-SA secondary antibody at 1:5000 with antibody diluent, add to the ELISA plate at 100 µL/well, incubate at room temperature for 30 min; wash five times with PBST, pat the ELISA plate dry on absorbent paper to remove excess liquid, add TMB substrate solution (purchased from KPL, catalog number: 5120-0075) at 100 µL/well, develop color to an appropriate intensity, add 2M H₂SO₄ at 50 µL/well to stop the color development, and measure the absorbance A at a wavelength of 450 nm using a multifunctional microplate reader, and analyze the data.

The calculation method for the IL-2 inhibition rate is: $IL - 2 inhibition rate \left(%\right) = \left(1-A\left〈sample\right〉/A\left〈negative control\right〉\right) \times 100 % .$

As shown in FIG. 12, TIGIT-CTLA4-FcG4-Enhl can dose-dependently inhibit the secretion of IL-2 by DC-induced CD4+ T cells, and in this experiment, when the concentration range was above 4.89 nM, the inhibitory activity of TIGIT-CTLA4-FcG4-Enhl was significantly superior to that of equimolar concentrations of the TIGIT and CTLA4 single fusion proteins, as well as the combination of the two single fusion proteins.

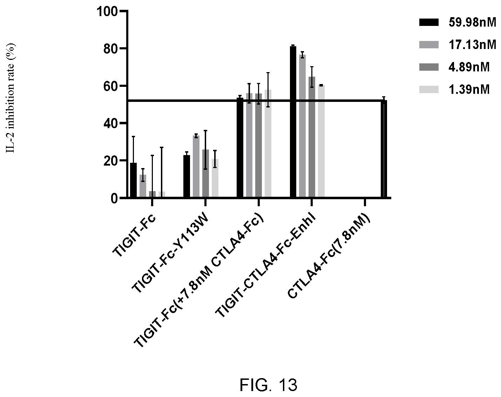

As shown in FIG. 13, TIGIT-CTLA4-FcG4-Enhl can dose-dependently inhibit the secretion of IL-2 by DC-induced CD8+ T cells, and in this experiment, when the concentration range was above 1.39 nM, the inhibitory activity of TIGIT-CTLA4-FcG4-Enhl was significantly superior to that of equimolar concentrations of the TIGIT and CTLA4 single fusion proteins, as well as the combination of the two single fusion proteins (7.8 nM is the concentration for the maximum inhibitory activity of CTLA4-Fc).

From the above experimental results, it can be seen that the bispecific fusion protein TIGIT-CTLA4-FcG4-Enhl has an additive effect in inhibiting the activation of CD4+ and CD8+ T cells, specifically manifested as the inhibitory activity exceeding that of the TIGIT and CTLA4 single fusion proteins at the same concentration when the fusion protein concentration is above 4.89 nM; in addition, it was found that targeting TIGIT has a significantly stronger response on CD4+ T cells than on CD8+ T cells, which may be due to differences in T cell subsets for the two co-stimulatory pathways of CD155/CD226 and CD80/CD86/CD28, and this also seems to explain the additive effect of the TIGIT and CTLA4 bispecific fusion protein.

### Example 11 In Vivo Efficacy Study of the Fusion Protein in a Collagen-Induced Arthritis Model

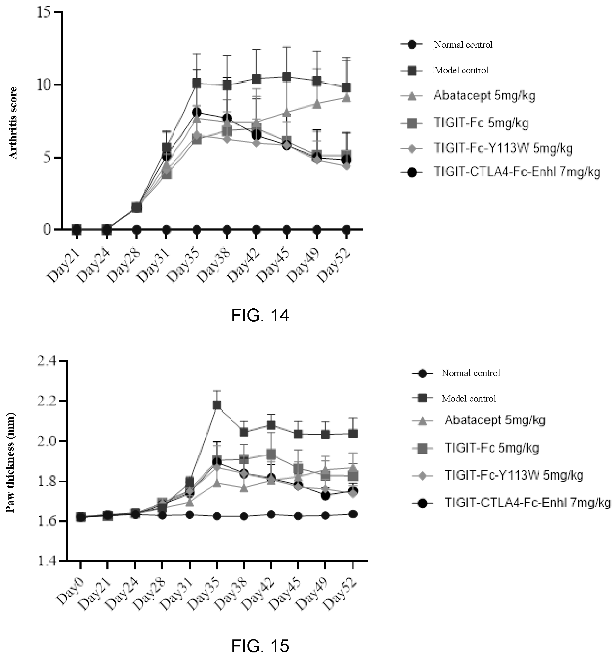

Collagen-induced arthritis (CIA) is an experimental autoimmune disease that can be induced by immunizing susceptible strains of rodents (rats and mice) with type II collagen. Immunized animals can develop an immune-mediated polyarthritis. Polyarthritis shares the same clinical, histological, and immunological features as human rheumatoid arthritis. In this example, 6-8 week old DBA/1 male mice (purchased from GemPharmatech Co., Ltd.) were used for modeling. On Day 0, an 8 mg/mL collagen solution (dissolved in 0.1 M acetic acid) was emulsified with an equal volume of complete Freund's adjuvant (purchased from Sigma) using a high-speed homogenizer. A single subcutaneous injection of the collagen emulsion was administered at the base of the tail of the mice in the modeling group. Three weeks later, i.e., on Day 21, an equal amount of the collagen emulsion as on Day 1 was injected again at the base of the tail. Seven days after the second immunization (Day 28), the mice in the modeling group were evenly divided into 5 groups based on arthritis scores, including one model control group (receiving vehicle) and four treatment groups, with 7 mice per group. Additionally, 7 mice that were not immunized with the collagen emulsion were selected and given vehicle as a normal control group. Dosing was initiated on the day of grouping, with intraperitoneal injections administered three times per week. TIGIT-Fc, TIGIT-Fc-Y113W, and the control drug Abatacept (sequence provided below) were dosed at 5 mg/kg, while TIGIT-CTLA4-FcG4-Enhl (hereinafter referred to as TIGIT-CTLA4-Fc-Enhl) was dosed at 7 mg/kg. Starting from the second immunization, the arthritis scores of the limbs of each animal in each group were observed twice a week until the end of the experiment. The scoring criteria were as follows: 0, no swelling; 1, swelling of 1-2 interphalangeal joints or slight swelling of the midfoot (tarsus) or ankle joint; 2, swelling of 3-4 interphalangeal joints or slight swelling from the ankle joint to the midfoot (tarsus); 3, moderate swelling from the ankle joint to the metatarsal joints; 4, severe swelling from the toes or fingers to the ankle or wrist joint. Before modeling on Day 0, the paw pad thickness of the hind paws of each animal was measured using a screw micrometer (purchased from Mitutoyo). Starting from Day 21, measurements were taken twice a week, and the paw pad thickness before and after immunization was compared. The arthritis scores and paw pad thickness measurement data were recorded and analyzed. The results are shown in FIG. 14 and FIG. 15. Compared with the normal control group, animals in the model control group and each drug treatment group developed significant arthritis symptoms, confirming successful modeling. Compared with the model control group, the arthritis symptoms of the animals in each group were significantly alleviated after drug treatment. Among them, after treatment with TIGIT-Fc-Y113W and TIGIT-CTLA4-Fc-Enhl, the arthritis symptoms in the limbs and the bilateral hind paw pad thickness of the animals recovered most significantly, which was superior to the TIGIT-Fc treatment group and the control drug Abatacept treatment group.

### Abatacept:

AMHVAQPAVVLASSRGIASFVCEYASPGKATEVRVTVLRQAD-
SQVTEVCAATYMMGNELT-
FLDDSICTGTSSGNQVNLTIQGLRAMDTGLYICKVELMYPPPYYLGIGNGTQI
YVIDPEPCPDSDQEPKSSDKTHTSPPSPAPELLGGSSVFLFPPKPKD-
TLMIS-
RTPEVTCVVVDVSHEDPEVKFNWYVDGVEVHNAKTKPREEQYNSTYRVVS
VLTVLHQDWLNGKEYKCKVSNKALPAPIEK-
TISKAKGQPREPQVYTLPPSRDELTKNQVSLTCLVKGFYPSDIAVEWESNG
QPENNYKTTPPVLDSDGSFFLYSKLTVDKSRWQQGNVFSCSVM-
HEALHNHYTQKSLSLSPGK

The present application relates to the following sequences:
SEQ ID NO. 1: Amino acid sequence of the CTLA4 extracellular domain (Ala37-Phe162) 
AMHVAQPAVVLASSRGIASFVCEYASPGKATEVRVTVLRQAD-
SQVTEVCAATYMMGNELT-
FLDDSICTGTSSGNQVNLTIQGLRAMDTGLYICKVELMYPPPYYLGIGNGTQI
YVIDPEPCPDSDF
SEQ ID NO. 2: Nucleic acid sequence of the CTLA4 extracellular domain (Ala37-Phe162) 
GCTATGCACGTGGCCCAGCCTGCCGTGGTGCTGGCTAGCTCTA-
GAGGCATTGCCTCTTTT-
GTGTGTGAGTACGCCTCTCCTGGCAAGGCTACTGAGGTGAGGGTGACA
GTGCTGAGACAGGCTGATTCTCAGGTGACAGAGGTGTGTGCTGCTAC-
CTATATGATGGG-
CAATGAGCTGACCTTTCTGGATGACTCCATCTGTACCGGCACCTCTTCTG
GCAACCAGGTGAACCTGACTATCCAGGGACTGAGAGCTATGGATA-
CAGGCCTGTATATCTG-
TAAGGTGGAGCTGATGTACCCACCTCCTTATTATCTGGGAATCGGTAACG
GAACCCAGATCTACGTGATCGATCCTGAGCCATGTCCTGATTCTGATTTT
SEQ ID NO. 3: Amino acid sequence of the TIGIT extracellular domain (Met22-Pro141)

MMTGTIETTGNISAEKGGSI*I*LQCHLSSTTAQVT*Q*VNWEQQDQLLAIC-
*N*AD*LG*WHISPSFKDRVAPGPGLGLTLQSLTVNDTGEYFCIYHT*Y*PDGTYTG
RIFLEVLESSVAEHGARFQIP

### Mutation positions are indicated in bold, italicized, and underlined: I42, Q56, N70, L73, G74, Y113.

SEQ ID NO. 4: Nucleic acid sequence of the TIGIT extracellular domain (Met22-Pro141) 
ATGATGACCGGCACAATCGAGACAACCGGTAATATCAGCGCTGA-
GAAGGGAG-
GATCCATCATCCTGCAGTGTCACCTGTCTAGTACTACAGCTCAGGTGACA
CAGGTGAACTGGGAGCAGCAGGATCAGCTGCTGGCTATCTG-
TAATGCTGATCTGGGATGG-
CATATTTCCCCTTCTTTTAAGGATAGAGTGGCTCCTGGACCTGGCCTGGG
CCTGACACTGCAGTCTCTGACCGTGAACGATactGGAGAGTATTTTT-
GCATCTATCATACAT-
ATCCTGATGGTACCTATACCGGAAGAATCTTTCTGGAGGTGCTGGAGTCC
TCTGTGGCTGAGCATGGAGCTAGATTTCAGATCCCT
SEQ ID NO. 5: Amino acid sequence of the CD28 extracellular domain (Asn19-Pro152) 
NKILVKQSPMLVAYDNAVNLSCKYSYNLFSREFRASLHKGLD-
SAVEVCVVYG-
NYSQQLQVYSKTGFNCDGKLGNESVTFYLQNLYVNQTDIYFCKIEVMYPPP
YLDNEKSNGTIIHVKGKHLCPSPLFPGPSKP
SEQ ID NO. 6: Amino acid sequence of the CD226 extracellular domain (Glu19-Asn247) 
EEVLWHTSVPFAENMSLECVYPSMGILTQVEWFKIGTQQDSIAIFSPTHGM-
VIRKPY-
AERVYFLNSTMASNNMTLFFRNASEDDVGYYSCSLYTYPQGTWQKVIQVV
 
QSDSFEAAVPSNSHIVSEPGKNVTLTCQPQMTWPVQAVRWE-
KIQPRQIDLLTYCNLVHGRN-
FTSKFPRQIVSNCSHGRWSVIVIPDVTVSDSGLYRCYLQASAGENETFVMR
LTVAEGKTDN
SEQ ID NO. 7: Amino acid sequence of IgG1-Fc 
EPKSCDKTHTCPPCPAPELLGGPSVFLFPPKPKDTLMIS-
RTPEVTCVVVDVSHEDPEVKFNWYVDGVEVHNAKTKPREEQYNSTYRVVS
VLTVLHQDWLNGKEYKCKVSNKALPAPIEK-
TISKAKGQPREPQVYTLPPSREEMTKNQVSLTCLVKGFYPSDIAVEWESNG
QPENNYKTTPPVLDSDGSFFLYSKLTVDKSRWQQGNVFSCSVM-
HEALHNHYTQKSLSLSPGK
SEQ ID NO. 8: Amino acid sequence of IgG4-Fc (S228P) 
ESKYGPPCPPCPAPEFLGGPSVFLFPPKPKDTLMIS-
RTPEVTCVVVDVSQEDPEVQFNWY-
VDGVEVHNAKTKPREEQFNSTYRVVSVLTVLHQDWLNGKEYKCKVSNKGL
PSSIEKTISKAKGQPREPQVYTLPPSQEEMTKNQVSLTCLVKGFYPSDI-
AVEWESNGQPEN-
NYKTTPPVLDSDGSFFLYSRLTVDKSRWQEGNVFSCSVMHEALHNHYTQK
SLSLSLGK
SEQ ID NO. 9: Amino acid sequence of CTLA4-LEAY 
AMHVAQPAVVLASSRGIASFVCEYASPGKYTEVRVTVLRQAD-
SQVTEVCAATYMMGNELT-
FLDDSICTGTSSGNQVNLTIQGLRAMDTGLYICKVELMYPPPYYEGIGNGTQ
IYVIDPEPCPDSDF
SEQ ID NO. 10: Nucleic acid sequence of CTLA4-LEAY 
GCTATGCACGTGGCCCAGCCTGCCGTGGTGCTGGCTAGCTCTA-
GAGGCATTGCCTCTTTT-
GTGTGTGAGTACGCCTCTCCTGGCAAGTATACTGAGGTGAGGGTGACAG
TGCTGAGACAGGCTGATTCTCAGGTGACAGAGGTGTGTGCTGCTAC-
CTATATGATGGG-
CAATGAGCTGACCTTTCTGGATGACTCCATCTGTACCGGCACCTCTTCTG
 
GCAACCAGGTGAACCTGACTATCCAGGGACTGAGAGCTATGGATA-
CAGGCCTGTATATCTG-
TAAGGTGGAGCTGATGTACCCACCTCCTTATTATGAGGGAATCGGTAACG
GAACCCAGATCTACGTGATCGATCCTGAGCCATGTCCTGATTCTGATTTT
SEQ ID NO. 11: Amino acid sequence of the CD86 extracellular domain (Leu20-Asp238) 
LKIQAYFNETADLPCQFANSQNQSLSELVVFWQDQENLVLNEVYL-
GKEKFDSVH-
SKYMGRTSFDSDSWTLRLHNLQIKDKGLYQCIIHHKKPTGMIRIHQMNSELS
VLANFSQPEIVPISNITENVYINLTCSSIHGYPEPKKMSVLLRTKN-
STIEYDGVMQKSQDNVTELYDVSISLSVSFPDVTSNMTIFCILETDKTRLLSS
PFSIELEDPQPPPD
SEQ ID NO. 12: Amino acid sequence of the CD155 extracellular domain (Gly27-Asn343) 
GDVVVQAPTQVPGFLGDSVTLPCYLQVPNMEVTHVSQLT-
WARHGESGSMAVFHQTQGPSYSESKRLEFVAARLGAELRNASLRMFGLRV
EDEGNYTCLFVTFPQGSRSVDIWLRVLAKPQNTAEVQKVQLTGEPVP-
MARCVSTGGRP-
PAQITWHSDLGGMPNTSQVPGFLSGTVTVTSLWILVPSSQVDGKNVTCKVE
HESFEKPQLLTVNLTVYYPPEVSISGYDNNWYLGQNEATLTCDARSNPEP-
TGYNWSTTMG-
PLPPFAVAQGAQLLIRPVDKPINTTLICNVTNALGARQAELTVQVKEGPPSEH
SGISRN
SEQ ID NO. 13: Amino acid sequence of CTLA4-Fc-TIGIT 
AMHVAQPAVVLASSRGIASFVCEYASPGKATEVRVTVLRQAD-
SQVTEVCAATYMMGNELT-
FLDDSICTGTSSGNQVNLTIQGLRAMDTGLYICKVELMYPPPYYLGIGNGTQI
YVIDPEPCPDSDFEPKSCDKTHTCPPCPAPELLGGPSVFLFPPKPKD-
TLMIS-
RTPEVTCVVVDVSHEDPEVKFNWYVDGVEVHNAKTKPREEQYNSTYRVVS
VLTVLHQDWLNGKEYKCKVSNKALPAPIEK-
TISKAKGQPREPQVYTLPPSREEMTKNQVSLTCLVKGFYPSDIAVEWESNG
 
QPENNYKTTPPVLDSDGSFFLYSKLTVDKSRWQQGNVFSCSVM-
HEALHNHYTQKSLSL-
SPGKGGGGSGGGGSGGGGSMMTGTIETTGNISAEKGGSIILQCHLSSTTA
QVTQVNWEQQDQLLAICNADLGWHISPSFKDRVAPGPGLGLT-
LQSLTVNDTGEYFCIYHTYPDGTYTGRIFLEVLESSVAEHGARFQIP
SEQ ID NO. 14: Nucleic acid sequence of CTLA4-Fc-TIGIT 
GCCATGCATGTGGCCCAGCCAGCTGTGGTGCTGGCTTCTAG-
TAGAGGTATTGCCAGCTTT-
GTGTGTGAGTATGCTAGTCCTGGAAAGGCCACAGAAGTGAGAGTGACC
GTGCTGAGGCAGGCTGATTCTCAGGTGACTGAAGTGTGTGCTGC-
TACTTATATGATGGG-
CAATGAGCTGACCTTTCTGGATGATTCTATTTGTACAGGCACTTCTAGCG
GCAATCAGGTGAATCTGACTATTCAGGGACTGAGAGCCATGGATA-
CAGGCTTGTATATCTG-
TAAAGTTGAACTGATGTATCCACCTCCCTATTATCTCGGAATCGGTAATGG
CACCCAGATCTACGTGATTGATCCAGAGCCCTGCCCTGACAGTGATTTT-
GAACCAAAGAGTT-
GTGATAAGACACACACATGTCCACCTTGTCCCGCTCCTGAGCTGCTGGG
CGGACCTAGCGTGTTTCTGTTTCCTCCAAAGCCTAAGGA-
TACCCTGATGATCTCTAG-
GACCCCTGAGGTGACCTGTGTGGTGGTGGATGTGAGCCATGAAGATCC
TGAGGTTAAATTCAATTGGTATGTGGATGGAGTGGAAGTG-
CATAATGCTAAGACCAAGCCTA-
GAGAAGAGCAGTATAATTCTACCTACCGCGTGGTGTCTGTCCTGACCGT
GCTGCATCAGGATTGGCTTAACGGAAAGGAATATAAATGTAAGGTGAG-
TAACAAGGCTCTCCCTGCTCCTATTGAAAAAACAATTTCTAAAGCTAAGG
GACAGCCTAGGGAGCCTCAGGTGTATACCTTGCCACCCTCTAGAGAG-
GAGATGACCAAGAAC-
CAGGTGAGCCTGACCTGCCTGGTGAAGGGCTTCTACCCCTCTGACATC
GCTGTGGAGTGGGAGAGCAACGGCCAGCCCGAGAACAACTACAAGAC-
CACCCCCCCTGTGCTGGACAGCGACGGCAGCTTCTTCCTGTACTCCAA
 
GCTGACCGTGGACAAGTCCAGGTGGCAGCAGGGCAAC-
GTGTTCAGCTGCTCCGTGATGCATGAGGCCCTGCACAACCACTACACCC
AGAAGAGCCTGAGCCTGTCCCCCGGCAAGGGCGGCGG-
CAGCGGCGGCGGCGGCTCCGGCGGCGGGGGCTCCATGATGACCGGC
ACCATCGAGACCACCGGCAACATCAGCGCTGAGAAGGGCGGCAG-
CATCATCCTGCAG-
TGCCACCTGAGCTCTACCACCGCCCAGGTGACCCAGGTGAACTGGGAG
CAACAGGACCAGCTGCTGGCCATCTGTAACGCCGACCTGGGCTGG-
CACATCTCTCCTAGCTTTAAGGACAGAGTGGCCCCCGGCCCCGGCCTG
GGCCTGACCCTGCAGTCCCTGACCGTGAACGACACCGGCGAG-
TACTTCTGCATCTACCACAC-
CTACCCCGACGGCACCTACACCGGCCGGATCTTCCTGGAGGTGCTGGA
GAGCAGCGTGGCCGAGCACGGCGCCAGGTTCCAGATCCCT
SEQ ID NO. 15: Amino acid sequence of CTLA4-TIGIT-Fc 
AMHVAQPAVVLASSRGIASFVCEYASPGKATEVRVTVLRQAD-
SQVTEVCAATYMMGNELT-
FLDDSICTGTSSGNQVNLTIQGLRAMDTGLYICKVELMYPPPYYLGIGNGTQI
YVIDPEPCPDSDFGGGGSGGGGSGGGGSMMTGTIETTGNISAEKGG-
SIILQCHLSSTTAQVTQVNWEQQDQLLAICNADLGWHISPSFKDRVAPGPGL
GLTLQSLTVNDTGEYFCIYHTYPDGTYTGRIFLEVLESSVAEHGAR-
FQIPEPKSCDKTHTCPPCPAPELLGGPSVFLFPPKPKDTLMISRTPEVTCVV
VDVSHEDPEVKFNWYVDGVEVHNAK-
TKPREEQYNSTYRVVSVLTVLHQDWLNGKEYKCK-
VSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVF-
SCSVMHEALHNHYTQKSLSLSPGK
SEQ ID NO. 16: Nucleic acid sequence of CTLA4-TIGIT-Fc 
GCTATGCACGTGGCCCAGCCTGCCGTGGTGCTGGCTAGCTCTA-
GAGGCATTGCCTCTTTT-
GTGTGTGAGTACGCCTCTCCTGGCAAGGCTACTGAGGTGAGGGTGACA
 
GTGCTGAGACAGGCTGATTCTCAGGTGACAGAGGTGTGTGCTGCTAC-
CTATATGATGGG-
CAATGAGCTGACCTTTCTGGATGACTCCATCTGTACCGGCACCTCTTCTG
GCAACCAGGTGAACCTGACTATCCAGGGACTGAGAGCTATGGATA-
CAGGCCTGTATATCTG-
TAAGGTGGAGCTGATGTACCCACCTCCTTATTATCTGGGAATCGGTAACG
GAACCCAGATCTACGTGATCGATCCTGAGCCATGTCCTGATTCTGATTTT-
GGTGGCGGCG-
GAAGCGGAGGTGGCGGCTCCGGCGGGGGCTCCATGATGACCGGC
ACAATCGAGACAACCGGTAATATCAGCGCTGAGAAGGGAG-
GATCCATCATCCTGCAG-
TGTCACCTGTCTAGTACTACAGCTCAGGTGACACAGGTGAACTGGGAGC
AGCAGGATCAGCTGCTGGCTATCTGTAATGCTGATCTGGGATGG-
CATATTTCCCCTTCTTTTAAGGATAGAGTGGCTCCTGGACCTGGCCTGGG
CCTGACACTGCAGTCTCTGACCGTGAACGATACTGGAGAGTATTTTT-
GCATCTATCATACAT-
ATCCTGATGGTACCTATACCGGAAGAATCTTTCTGGAGGTGCTGGAGTCC
TCTGTGGCTGAGCATGGAGCTAGATTTCAGATCCCTGAGCCCAAGTCTT-
GTGA-
TAAAACACATACATGCCCTCCATGTCCAGCTCCTGAACTGCTGGGCGGC
CCATCTGTGTTCCTGTTTCCACCAAAGCCTAAGGACACAC-
TGATGATCTCTAGAACAC-
CTGAAGTGACTTGTGTGGTGGTGGATGTGTCTCATGAAGATCCTGAGGT
GAAGTTTAATTGGTATGTGGATGGCGTGGAGGTGCATAATGCTAAAAC-
CAAGCCAAGGGAG-
GAGCAGTACAACTCTACTTATAGGGTGGTGTCAGTGCTGACTGTGCTGC
ACCAGGATTGGCTGAATGGAAAGGAATATAAGTGTAAAGTGAG-
TAACAAA-
GCTCTGCCTGCTCCAATCGAAAAGACAATCTCTAAGGCTAAGGGCCAGC
CTAGAGAACCACAGGTGTATACACTGCCACCTAGCAGAGAAGAGATGAC-
CAA-
GAATCAGGTGTCTCTGACCTGCCTGGTGAAAGGCTTTTATCCTTCTGACA
 
TCGCCGTGGAGTGGGAGTCTAACGGCCAGCCTGAGAATAATTATAAGA-
CAACCCCTCCTGTGCTGGATTCTGATGGCTCATTCTTTCTGTACTCTAAG
CTGACAGTGGATAAGTCTAGATGGCAGCAGGGTAATGTGTTTTCTT-
GTAG-
TGTGATGCATGAGGCTCTGCATAACCATTATACCCAGAAGTCTCTGAGTC
TGAGCCCCGGCAAG
SEQ ID NO. 17: Amino acid sequence of TIGIT-CTLA4-Fc 
MMTGTIETTGNISAEKGGSIILQCHLSSTTAQVTQVNWEQQDQLLAIC-
NADLGWHISPSFKDRVAPGPGLGLTLQSLTVNDTGEYFCIYHTYPDGTYTG
RIFLEVLESSVAEHGARFQIPGGGGSGGGGSGGGGSAMHVAQPAV-
VLASSRGI-
ASFVCEYASPGKATEVRVTVLRQADSQVTEVCAATYMMGNELTFLDDSICT
GTSSGNQVNLTIQGLRAMDTGLYICKVELMYPPPYYL-
GIGNGTQIYVIDPEPCPDSD-
FEPKSCDKTHTCPPCPAPELLGGPSVFLFPPKPKDTLMISRTPEVTCVVVDV
SHEDPEVKFNWYVDGVEVHNAK-
TKPREEQYNSTYRVVSVLTVLHQDWLNGKEYKCKVSNKAL-
PAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVM-
HEALHNHYTQKSLSLSPGK
SEQ ID NO. 18: Nucleic acid sequence of TIGIT-CTLA4-Fc 
ATGATGACCGGCACAATCGAGACAACCGGTAATATCAGCGCTGA-
GAAGGGAG-
GATCCATCATCCTGCAGTGTCACCTGTCTAGTACTACAGCTCAGGTGACA
CAGGTGAACTGGGAGCAGCAGGATCAGCTGCTGGCTATCTG-
TAATGCTGATCTGGGATGG-
CATATTTCCCCTTCTTTTAAGGATAGAGTGGCTCCTGGACCTGGCCTGGG
CCTGACACTGCAGTCTCTGACCGTGAACGATACTGGAGAGTATTTTT-
GCATCTATCATACAT-
ATCCTGATGGTACCTATACCGGAAGAATCTTTCTGGAGGTGCTGGAGTCC
 
TCTGTGGCTGAGCATGGAGCTAGATTTCAGATCCCTGGTGGCGGCG-
GAAGCG-
GAGGTGGCGGCTCCGGCGGCGGGGGCTCCGCTATGCACGTGGCCCAG
CCTGCCGTGGTGCTGGCTAGCTCTAGAGGCATTGCCTCTTTT-
GTGTGTGAGTAC-
GCCTCTCCTGGCAAGGCTACTGAGGTGAGGGTGACAGTGCTGAGACAG
GCTGATTCTCAGGTGACAGAGGTGTGTGCTGCTACCTATATGATGGG-
CAATGAGCTGAC-
CTTTCTGGATGACTCCATCTGTACCGGCACCTCTTCTGGCAACCAGGTG
AACCTGACTATCCAGGGACTGAGAGCTATGGATACAGGCCTG-
TATATCTGTAAGGTG-
GAGCTGATGTACCCACCTCCTTATTATCTGGGAATCGGTAACGGAACCCA
GATCTACGTGATCGATCCTGAGCCATGTCCTGATTCTGATTTTGAAC-
CTAAGTCTTGTGA-
TAAGACCCATACCTGTCCTCCTTGTCCTGCCCCAGAGCTGCTGGGAGGC
CCTTCTGTGTTTCTGTTTCCTCCTAAGCCTAAAGA-
TACCCTGATGATCTCTA-
GAACTCCTGAGGTGACCTGTGTGGTGGTGGATGTGTCTCACGAGGATC
CTGAAGTGAAGTTTAACTGGTATGTGGATGGAGTGGAGGTGCATAAC-
GCTAAGACTAA-
GCCTAGAGAGGAGCAGTATAATAGCACTTATAGGGTGGTGAGCGTGCTG
ACCGTGCTGCATCAGGATTGGCTGAATGGCAAGGAGTATAAGTG-
TAAGGTGTCTAA-
TAAGGCTCTGCCAGCTCCTATCGAGAAAACCATCTCCAAGGCTAAGGGA
CAGCCTAGGGAGCCTCAGGTGTATACCCTGCCTCCTTCTAGAGAGGA-
GATGACAAA-
GAATCAGGTGTCCCTGACATGTCTGGTGAAGGGCTTTTACCCATCTGATA
TCGCTGTGGAGTGGGAGTCTAATGGCCAGCCTGAGAATAATTATAAGA-
CAACACCAC-
CTGTGCTGGATTCTGATGGTTCTTTCTTTCTGTATAGCAAGCTGACCGTG
 
GATAAGTCTAGATGGCAGCAGGGAAAC-
GTGTTTAGCTGTTCTGTGATGCATGAGGCTCTG-
CATAATCACTATACCCAGAAGTCCCTGTCTCTGAGCCCTGGAAAG
SEQ ID NO. 19: Amino acid sequence of TIGIT-Fc-CTLA4 
MMTGTIETTGNISAEKGGSIILQCHLSSTTAQVTQVNWEQQDQLLAIC-
NADLGWHISPSFKDRVAPGPGLGLTLQSLTVNDTGEYFCIYHTYPDGTYTG
RIFLEVLESSVAEHGARFQIPEPKSCDKTHTCPPCPAPELLGGPSVFLF-
PPKPKDTLMIS-
RTPEVTCVVVDVSHEDPEVKFNWYVDGVEVHNAKTKPREEQYNSTYRVVS
VLTVLHQDWLNGKEYKCKVSNKALPAPIEK-
TISKAKGQPREPQVYTLPPSREEMTKNQVSLTCLVKGFYPSDIAVEWESNG
QPENNYKTTPPVLDSDGSFFLYSKLTVDKSRWQQGNVFSCSVM-
HEALHNHYTQKSLSL-
SPGKGGGGSGGGGSGGGGSAMHVAQPAVVLASSRGIASFVCEYASPGKA
TEVRVTVLRQADSQVTEVCAATYMMGNELT-
FLDDSICTGTSSGNQVNLTIQGLRAMDTGLYICKVELMYPPPYYLGIGNGTQI
YVIDPEPCPDSDF
SEQ ID NO. 20: Nucleic acid sequence of TIGIT-Fc-CTLA4 
ATGATGACCGGCACAATCGAGACAACCGGTAATATCAGCGCTGA-
GAAGGGAG-
GATCCATCATCCTGCAGTGTCACCTGTCTAGTACTACAGCTCAGGTGACA
CAGGTGAACTGGGAGCAGCAGGATCAGCTGCTGGCTATCTG-
TAATGCTGATCTGGGATGG-
CATATTTCCCCTTCTTTTAAGGATAGAGTGGCTCCTGGACCTGGCCTGGG
CCTGACACTGCAGTCTCTGACCGTGAACGATACTGGAGAGTATTTTT-
GCATCTATCATACAT-
ATCCTGATGGTACCTATACCGGAAGAATCTTTCTGGAGGTGCTGGAGTCC
TCTGTGGCTGAGCATGGAGCTAGATTTCAGATCCCTGAACCTAAGTCTT-
GTGATAA-
GACCCATACCTGTCCTCCTTGTCCTGCCCCAGAGCTGCTGGAGGCCC
TTCTGTGTTTCTGTTTCCTCCTAAGCCTAAAGATACCCTGATGATCTCTA-
GAACTCCTGAGGTGACCTGTGTGGTGGTGGATGTGTCTCACGAGGATC
 
CTGAAGTGAAGTTTAACTGGTATGTGGATGGAGTGGAGGTGCATAAC-
GCTAAGACTAA-
GCCTAGAGAGGAGCAGTATAATAGCACTTATAGGGTGGTGAGCGTGCTG
ACCGTGCTGCATCAGGATTGGCTGAATGGCAAGGAGTATAAGTG-
TAAGGTGTCTAA-
TAAGGCTCTGCCAGCTCCTATCGAGAAAACCATCTCCAAGGCTAAGGGA
CAGCCTAGGGAGCCTCAGGTGTATACCCTGCCTCCTTCTAGAGAGGA-
GATGACAAA-
GAATCAGGTGTCCCTGACATGTCTGGTGAAGGGCTTTTACCCATCTGATA
TCGCTGTGGAGTGGGAGTCTAATGGCCAGCCTGAGAATAATTATAAGA-
CAACACCAC-
CTGTGCTGGATTCTGATGGTTCTTTCTTTCTGTATAGCAAGCTGACCGTG
GATAAGTCTAGATGGCAGCAGGGAAAC-
GTGTTTAGCTGTTCTGTGATGCATGAGGCTCTG-
CATAATCACTATACCCAGAAGTCCCTGTCTCTGAGCCCTGGAAAGGGTG
GCGGCGGAAGCGGAGGTGGCGGCTCCGGCGGCGGGGGCTCCGC-
TATGCAC-
GTGGCCCAGCCTGCCGTGGTGCTGGCTAGCTCTAGAGGCATTGCCTCT
TTTGTGTGTGAGTACGCCTCTCCTGGCAAGGC-
TACTGAGGTGAGGGTGACAGTGCTGAGA-
CAGGCTGATTCTCAGGTGACAGAGGTGTGTGCTGCTACCTATATGATGG
GCAATGAGCTGACCTTTCTGGATGACTCCATCTGTACCGGCAC-
CTCTTCTGGCAAC-
CAGGTGAACCTGACTATCCAGGGACTGAGAGCTATGGATACAGGCCTGT
ATATCTGTAAGGTGGAGCTGATGTACCCACCTCCTTATTATCTGG-
GAATCGGTAAC-
GGAACCCAGATCTACGTGATCGATCCTGAGCCATGTCCTGATTCTGATTT
T
SEQ ID NO. 21: Amino acid sequence of TIGIT extracellular domain-Y113W 
MMTGTIETTGNISAEKGGSIILQCHLSSTTAQVTQVNWEQQDQLLAIC-
NADLGWHISPSFKDRVAPGPGLGLTLQSLTVNDTGEYFCIYHTWPDGTYTG
RIFLEVLESSVAEHGARFQIP
SEQ ID NO. 22: Nucleic acid sequence of TIGIT extracellular domain-Y113W 
ATGATGACCGGCACAATCGAGACAACCGGTAATATCAGCGCTGA-
GAAGGGAG-
GATCCATCATCCTGCAGTGTCACCTGTCTAGTACTACAGCTCAGGTGACA
CAGGTGAACTGGGAGCAGCAGGATCAGCTGCTGGCTATCTG-
TAATGCTGATCTGGGATGG-
CATATTTCCCCTTCTTTTAAGGATAGAGTGGCTCCTGGACCTGGCCTGGG
CCTGACACTGCAGTCTCTGACCGTGAACGATactGGAGAGTATTTTT-
GCATCTATCATA-
CATGGCCTGATGGTACCTATACCGGAAGAATCTTTCTGGAGGTGCTGGA
GTCCTCTGTGGCTGAGCATGGAGCTAGATTTCAGATCCCT
SEQ ID NO. 23: Amino acid sequence of CTLA4-FcG4-TIGIT-EnhI
AMHVAQPAVVLASSRGIASFVCEYASPGKYTEVRVTVLRQAD-
SQVTEVCAATYMMGNELT-
FLDDSICTGTSSGNQVNLTIQGLRAMDTGLYICKVELMYPPPYYEGIGNGTQ
IYVIDPEPCPDSDESKYGPPCPPCPAPEFLGGPSVFLFPPKPKDTLMIS-
RTPEVTCVVVDVSQEDPEVQFNWYVDGVEVHNAKTKPREEQFNSTYRVV
SVLTVLHQDWLNGKEYKCKVSNKGLPSSIEKTISKAKGQPREPQVYTLPP-
SQEEMTKNQVSLTCLVKGFYPSDIAVEWESNGQPENNYKTTPPVLDSDGS
FFLYSRLTVDKSRWQEGNVF-
SCSVLHEALHSHYTQKSLSLSLGKGGGGSGGGGSGGGGSMMTGTIETTG
NISAEKGGSIILQCHLSSTTAQVTQVNWEQQDQLLAIC-
NADLGWHISPSFKDRVAPGPGLGLTLQSLTVNDTGEYFCIYHTWPDGTYTG
RIFLEVLESSVAEHGARFQIP
SEQ ID NO. 24: Nucleic acid sequence of CTLA4-FcG4-TIGIT-Enhl 
GCTATGCACGTGGCCCAGCCTGCCGTGGTGCTGGCTAGCTCTA-
GAGGCATTGCCTCTTTT-
GTGTGTGAGTACGCCTCTCCTGGCAAGTATACTGAGGTGAGGGTGACAG
TGCTGAGACAGGCTGATTCTCAGGTGACAGAGGTGTGTGCTGCTAC-
CTATATGATGGG-
CAATGAGCTGACCTTTCTGGATGACTCCATCTGTACCGGCACCTCTTCTG
 
GCAACCAGGTGAACCTGACTATCCAGGGACTGAGAGCTATGGATA-
CAGGCCTGTATATCTG-
TAAGGTGGAGCTGATGTACCCACCTCCTTATTATGAGGGAATCGGTAACG
GAACCCAGATCTACGTGATCGATCCTGAGCCATGTCCTGAT-
TCTGATGAATCTAAA-
TATGGCCCTCCATGCCCACCTTGTCCTGCCCCTGAGTTTCTGGGAGGCC
CTAGCGTGTTTCTGTTTCCACCAAAACCTAAGGATACAC-
TGATGATCTCTAGGACAC-
CTGAAGTGACATGTGTGGTAGTGGATGTGTCCCAGGAAGATCCTGAGGT
GCAGTTTAATTGGTATGTGGATGGCGTGGAAGTGCACAAC-
GCTAAAACTAAGCCTAGGGAG-
GAGCAGTTTAATTCCACATATAGAGTGGTGTCTGTGCTGACCGTGCTGCA
TCAGGATTGGCTGAATGGCAAGGAATACAAGTGTAAGGTGTCTAA-
TAAGGGCCTGCCTTCCTCCATTGAAAAGACCATTTCTAAAGCTAAGGGTC
AGCCTAGGGAGCCTCAGGTGTACACCCTGCCTCCTAGCCAGGAA-
GAAATGACTAA-
GAATCAGGTGAGTCTGACTTGCCTGGTGAAGGGCTTCTACCCTTCTGAC
ATTGCTGTGGAGTGGGAGTCTAATGGACAGCCTGAGAATAATTATAA-
GAC-
CACCCCCCCTGTGCTGGATTCTGATGGTAGCTTCTTTCTGTATTCTAGAC
TGACCGTGGATAAGTCTAGATGGCAGGAGGGAAACGTGTTTTCTT-
GTTCTGTGCTGCATGAA-
GCTCTCCACAGCCATTACACCCAAAAGTCCCTCTCCCTGAGCCTAGGTA
AAGGTGGAGGCGGATCTGGCGGTGGCGGAAGTGGGGGTGGAG-
GATCAATGATGACCGG-
CACAATCGAGACAACCGGTAATATCAGCGCTGAGAAGGGAGGATCCATC
ATCCTGCAGTGTCACCTGTCTAGTACTA-
CAGCTCAGGTGACACAGGTGAACTGGGAGCAG-
CAGGATCAGCTGCTGGCTATCTGTAATGCTGATCTGGGATGGCATATTTC
CCCTTCTTTTAAGGATAGAGTGGCTCCTGGAC-
CTGGCCTGGGCCTGACACTGCAG-
TCTCTGACCGTGAACGATactGGAGAGTATTTTTGCATCTATCATACATGGC
 
CTGATGGTACCTATACCGGAAGAATCTTTCTGGAGGTGCTGGAG-
TCCTCTGTGGCTGAGCATGGAGCTAGATTTCAGATCCCT
SEQ ID NO. 25: Amino acid sequence of CTLA4-TIGIT-FcG4-Enhl 
AMHVAQPAVVLASSRGIASFVCEYASPGKYTEVRVTVLRQAD-
SQVTEVCAATYMMGNELT-
FLDDSICTGTSSGNQVNLTIQGLRAMDTGLYICKVELMYPPPYYEGIGNGTQ
IYVIDPEPCPDSDGGGGSGGGGSGGGGSMMTGTIETTGNISAEKGG-
SIILQCHLSSTTAQVTQVNWEQQDQLLAICNADLGWHISPSFKDRVAPGPGL
GLTLQSLTVNDTGEYFCIYHTWPDGTYTGRIFLEVLESSVAEHGAR-
FQIPESKYGPPCPPCPAPEFLGGPSVFLFPPKPKDTLMISRTPEVTCVVVDV
SQEDPEVQFNWYVDGVEVHNAK-
TKPREEQFNSTYRVVSVLTVLHQDWLNGKEYKCK-
VSNKGLPSSIEKTISKAKGQPREPQVYTLPPSQEEMTKNQVSLTCLVKGFYP
SDIAVEWESNGQPENNYKTTPPVLDSDGSFFLYSRLTVDKSRWQEGNVF-
SCSVLHEALHSHYTQKSLSLSLGK
SEQ ID NO. 26: Nucleic acid sequence of CTLA4-TIGIT-FcG4-Enhl 
GCTATGCACGTGGCCCAGCCTGCCGTGGTGCTGGCTAGCTCTA-
GAGGCATTGCCTCTTTT-
GTGTGTGAGTACGCCTCTCCTGGCAAGTATACTGAGGTGAGGGTGACAG
TGCTGAGACAGGCTGATTCTCAGGTGACAGAGGTGTGTGCTGCTAC-
CTATATGATGGG-
CAATGAGCTGACCTTTCTGGATGACTCCATCTGTACCGGCACCTCTTCTG
GCAACCAGGTGAACCTGACTATCCAGGGACTGAGAGCTATGGATA-
CAGGCCTGTATATCTG-
TAAGGTGGAGCTGATGTACCCACCTCCTTATTATGAGGGAATCGGTAACG
GAACCCAGATCTACGTGATCGATCCTGAGCCATGTCCTGAT-
TCTGATGGTGGAGGCG-
GATCTGGCGGTGGCGGAAGTGGGGGTGGAGGATCAATGATGACCGGCA
CAATCGAGACAACCGGTAATATCAGCGCTGAGAAGGGAG-
GATCCATCATCCTGCAGTGTCAC-
CTGTCTAGTACTACAGCTCAGGTGACACAGGTGAACTGGGAGCAGCAG
 
GATCAGCTGCTGGCTATCTGTAATGCTGATCTGGGATGG-
CATATTTCCCCTTCTTTTAAGGA-
TAGAGTGGCTCCTGGACCTGGCCTGGGCCTGACACTGCAGTCTCTGAC
CGTGAACGATactGGAGAGTATTTTTGCATCTATCATACATGGCCTGATGG-
TACCTATACCG-
GAAGAATCTTTCTGGAGGTGCTGGAGTCCTCTGTGGCTGAGCATGGAG
CTAGATTTCAGATCCCTGAATCTAAATATGGCCCTCCATGCCCACCTT-
GTCCTGCCCCTGAG-
TTTCTGGGAGGCCCTAGCGTGTTTCTGTTTCCACCAAAACCTAAGGATAC
ACTGATGATCTCTAGGACACCTGAAGTGACATGTGTGGTAGTG-
GATGTGTCCCAGGAA-
GATCCTGAGGTGCAGTTTAATTGGTATGTGGATGGCGTGGAAGTGCACA
ACGCTAAAACTAAGCCTAGGGAGGAGCAGTTTAATTCCACATATAGAG-
TGGTGTCTGTGCTGACCGTGCTGCATCAGGATTGGCTGAATGGCAAGGA
ATACAAGTGTAAGGTGTCTAATAAGGGCCTGCCTTCCTCCATTGAAAA-
GACCATTTCTAAA-
GCTAAGGGTCAGCCTAGGGAGCCTCAGGTGTACACCCTGCCTCCTAGC
CAGGAAGAAATGACTAAGAATCAGGTGAGTCTGACTT-
GCCTGGTGAAGGGCTTC-
TACCCTTCTGACATTGCTGTGGAGTGGGAGTCTAATGGACAGCCTGAGA
ATAATTATAAGACCACCCCCCCTGTGCTGGATTCTGATGG-
TAGCTTCTTTCTGTATTCTA-
GACTGACCGTGGATAAGTCTAGATGGCAGGAGGGAAACGTGTTTTCTTG
TTCTGTGCTGCATGAA-
GCTCTCCACAGCCATTACACCCAAAAGTCCCTCTCCCTGAGCCTAGGTA
AA
SEQ ID NO. 27: Amino acid sequence of TIGIT-CTLA4-FcG4-Enhl 
MMTGTIETTGNISAEKGGSIILQCHLSSTTAQVTQVNWEQQDQLLAIC-
NADLGWHISPSFKDRVAPGPGLGLTLQSLTVNDTGEYFCIYHTWPDGTYTG
RIFLEVLESSVAEHGARFQIPGGGGSGGGGSGGGGSAMHVAQPAV-
VLASSRGI-
ASFVCEYASPGKYTEVRVTVLRQADSQVTEVCAATYMMGNELTFLDDSICT
 
GTSSGNQVNLTIQGLRAMDTGLYICKVELMYPP-
PYYEGIGNGTQIYVIDPEPCPDSDESKYGPPCPPCPAPEFLGGPSVFLFPPK
PKDTLMISRTPEVTCVVVDVSQEDPEVQFNWYVDGVEVHNAK-
TKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPEN-
NYKTTPPVLDSDGSFFLYSRLTVDKSRWQEGNVFSCSVLHEALHSHYTQKS
LSLSLGK
SEQ ID NO. 28: Nucleic acid sequence of TIGIT-CTLA4-FcG4-Enhl 
ATGATGACCGGCACAATCGAGACAACCGGTAATATCAGCGCTGA-
GAAGGGAG-
GATCCATCATCCTGCAGTGTCACCTGTCTAGTACTACAGCTCAGGTGACA
CAGGTGAACTGGGAGCAGCAGGATCAGCTGCTGGCTATCTG-
TAATGCTGATCTGGGATGG-
CATATTTCCCCTTCTTTTAAGGATAGAGTGGCTCCTGGACCTGGCCTGGG
CCTGACACTGCAGTCTCTGACCGTGAACGATactGGAGAGTATTTTT-
GCATCTATCATA-
CATGGCCTGATGGTACCTATACCGGAAGAATCTTTCTGGAGGTGCTGGA
GTCCTCTGTGGCTGAGCATGGAGCTAGATTTCAGATCCCTGGTG-
GAGGCG-
GATCTGGCGGTGGCGGAAGTGGGGGTGGAGGATCAGCTATGCACGTG
GCCCAGCCTGCCGTGGTGCTGGCTAGCTCTAGAGGCATTGCCTCTTTT-
GTGTGTGAGTAC-
GCCTCTCCTGGCAAGTATACTGAGGTGAGGGTGACAGTGCTGAGACAG
GCTGATTCTCAGGTGACAGAGGTGTGTGCTGCTACCTATATGATGGG-
CAATGAGCTGAC-
CTTTCTGGATGACTCCATCTGTACCGGCACCTCTTCTGGCAACCAGGTG
AACCTGACTATCCAGGGACTGAGAGCTATGGATACAGGCCTG-
TATATCTGTAAGGTG-
GAGCTGATGTACCCACCTCCTTATTATGAGGGAATCGGTAACGGAACCCA
GATCTACGTGATCGATCCTGAGCCATGTCCTGATTCTGATGAATCTAAA-
TATGGCCCTCCATGCCCACCTTGTCCTGCCCCTGAGTTTCTGGGAGGCC
 
CTAGCGTGTTTCTGTTTCCACCAAAACCTAAGGATACAC-
TGATGATCTCTAGGACAC-
CTGAAGTGACATGTGTGGTAGTGGATGTGTCCCAGGAAGATCCTGAGGT
GCAGTTTAATTGGTATGTGGATGGCGTGGAAGTGCACAAC-
GCTAAAACTAAGCCTAGGGAG-
GAGCAGTTTAATTCCACATATAGAGTGGTGTCTGTGCTGACCGTGCTGCA
TCAGGATTGGCTGAATGGCAAGGAATACAAGTGTAAGGTGTCTAA-
TAAGGGCCTGCCTTCCTCCATTGAAAAGACCATTTCTAAAGCTAAGGGTC
AGCCTAGGGAGCCTCAGGTGTACACCCTGCCTCCTAGCCAGGAA-
GAAATGACTAA-
GAATCAGGTGAGTCTGACTTGCCTGGTGAAGGGCTTCTACCCTTCTGAC
ATTGCTGTGGAGTGGGAGTCTAATGGACAGCCTGAGAATAATTATAA-
GAC-
CACCCCCCCTGTGCTGGATTCTGATGGTAGCTTCTTTCTGTATTCTAGAC
TGACCGTGGATAAGTCTAGATGGCAGGAGGGAAACGTGTTTTCTT-
GTTCTGTGCTGCATGAA-
GCTCTCCACAGCCATTACACCCAAAAGTCCCTCTCCCTGAGCCTAGGTA
AA
SEQ ID NO. 29: Amino acid sequence of TIGIT-FcG4-CTLA4-Enhl 
MMTGTIETTGNISAEKGGSIILQCHLSSTTAQVTQVNWEQQDQLLAIC-
NADLGWHISPSFKDRVAPGPGLGLTLQSLTVNDTGEYFCIYHTWPDGTYTG
RIFLEVLESSVAEHGARFQIPESKYGPPCPPCPAPEFLGGPSVFLF-
PPKPKDTLMIS-
RTPEVTCVVVDVSQEDPEVQFNWYVDGVEVHNAKTKPREEQFNSTYRVV
SVLTVLHQDWLNGKEYKCKVSNKGLPSSIEKTISKAKGQPREPQVYTLPP-
SQEEMTKNQVSLTCLVKGFYPSDIAVEWESNGQPENNYKTTPPVLDSDGS
FFLYSRLTVDKSRWQEGNVF-
SCSVLHEALHSHYTQKSLSLSLGKGGGGSGGGGSGGGGSAM-
HVAQPAVVLASSRGIASFVCEYASPGKYTEVRVTVLRQADSQVTEVCAATY
MMGNELTFLDDSICTGTSSGNQVNLTIQGLRAMDTGLYICKVELMYPP-
PYYEGIGNGTQIYVIDPEPCPDSD
SEQ ID NO. 30: Nucleic acid sequence of TIGIT-FcG4-CTLA4-Enhl

ATGATGACCGGCACAATCGAGACAACCGGTAATATCAGCGCTGA-
GAAGGGAG-
GATCCATCATCCTGCAGTGTCACCTGTCTAGTACTACAGCTCAGGTGACA
CAGGTGAACTGGGAGCAGCAGGATCAGCTGCTGGCTATCTG-
TAATGCTGATCTGGGATGG-
CATATTTCCCCTTCTTTTAAGGATAGAGTGGCTCCTGGACCTGGCCTGGG
CCTGACACTGCAGTCTCTGACCGTGAACGATactGGAGAGTATTTTT-
GCATCTATCATA-
CATGGCCTGATGGTACCTATACCGGAAGAATCTTTCTGGAGGTGCTGGA
GTCCTCTGTGGCTGAGCATGGAGCTAGATTTCAGATCCCTGAATCTAAA-
TATGGCCCTCCATGCCCACCTTGTCCTGCCCCTGAGTTTCTGGGAGGCC
CTAGCGTGTTTCTGTTTCCACCAAAACCTAAGGATACAC-
TGATGATCTCTAGGACAC-
CTGAAGTGACATGTGTGGTAGTGGATGTGTCCCAGGAAGATCCTGAGGT
GCAGTTTAATTGGTATGTGGATGGCGTGGAAGTGCACAAC-
GCTAAAACTAAGCCTAGGGAG-
GAGCAGTTTAATTCCACATATAGAGTGGTGTCTGTGCTGACCGTGCTGCA
TCAGGATTGGCTGAATGGCAAGGAATACAAGTGTAAGGTGTCTAA-
TAAGGGCCTGCCTTCCTCCATTGAAAAGACCATTTCTAAAGCTAAGGGTC
AGCCTAGGGAGCCTCAGGTGTACACCCTGCCTCCTAGCCAGGAA-
GAAATGACTAA-
GAATCAGGTGAGTCTGACTTGCCTGGTGAAGGGCTTCTACCCTTCTGAC
ATTGCTGTGGAGTGGGAGTCTAATGGACAGCCTGAGAATAATTATAA-
GAC-
CACCCCCCCTGTGCTGGATTCTGATGGTAGCTTCTTTCTGTATTCTAGAC
TGACCGTGGATAAGTCTAGATGGCAGGAGGGAAACGTGTTTTCTT-
GTTCTGTGCTGCATGAA-
GCTCTCCACAGCCATTACACCCAAAAGTCCCTCTCCCTGAGCCTAGGTA
AAGGTGGAGGCGGATCTGGCGGTGGCGGAAGTGGGGGTGGAG-
GATCAGCTATGCAC-
GTGGCCCAGCCTGCCGTGGTGCTGGCTAGCTCTAGAGGCATTGCCTCT

TTTGTGTGTGAGTACGCCTCTCCTGG-
CAAGTATACTGAGGTGAGGGTGACAGTGCTGAGA-
CAGGCTGATTCTCAGGTGACAGAGGTGTGTGCTGCTACCTATATGATGG
GCAATGAGCTGACCTTTCTGGATGACTCCATCTGTACCGGCAC-
CTCTTCTGGCAAC-
CAGGTGAACCTGACTATCCAGGGACTGAGAGCTATGGATACAGGCCTGT
ATATCTGTAAGGTGGAGCTGATGTACCCAC-
CTCCTTATTATGAGGGAATCGGTAAC-
GGAACCCAGATCTACGTGATCGATCCTGAGCCATGTCCTGATTCTGAT

All publications mentioned in this application are incorporated herein by reference, as if each individual publication was specifically and individually indicated to be incorporated by reference. It should also be understood that, after reading the above teachings of the present application, those skilled in the art can make various changes or modifications to the present application, and these equivalent forms also fall within the scope defined by the appended claims of the present application.

### Industrial Applicability

Through multiple rounds of protein engineering and screening, the present application has obtained a bispecific fusion protein CTLA4-TIGIT that can competitively inhibit the activation of the CD28-CD80/86 and CD226-CD155 pathways, exerting a synergistic inhibitory effect on the activation of CD4⁺ and CD8⁺ T cells that surpasses that of CTLA4 or TIGIT fusion proteins used alone, and will exert more effective and broader therapeutic effects in related autoimmune diseases.

Through extensive research and analysis, the present application has identified key amino acid sites that affect the stability of the "LOCK-and-KEY" interface of the TIGIT/CD155 interaction. On this basis, TIGIT polypeptide variants with significantly enhanced affinity and slower dissociation kinetics were obtained through multiple rounds of screening, thereby achieving unexpected technical advantages. The TIGIT polypeptide variants have superior biological activities, including a stronger ability to block the CD226/CD155 signaling pathway and the ability to exert stable immunomodulatory activity at lower doses. When used in the clinical treatment of immune system diseases, benefits can be obtained in multiple aspects, including efficacy, safety, stability, and durability.

The present application provides a new dual-target combination scheme, constructing a novel fusion protein by combining the TIGIT extracellular domain protein and the CTLA4 extracellular domain protein for the first time.

The TIGIT/CTLA4 fusion protein of the present application can better inhibit T cell activation/proliferation and inflammatory cytokine secretion by simultaneously blocking the CD226/CD155 and CD80/CD86-CD28 signaling pathways. This addresses clinical problems that are unattainable with monotherapy or combination therapy. As demonstrated by the exemplary TIGIT/CTLA4 fusion protein, it possesses superior signaling pathway inhibition ability and T cell inhibitory activity compared to each single-target fusion protein, and its T cell inhibitory activity is superior to the combination of TIGIT fusion protein and CTLA4 fusion protein, exhibiting unexpected synergistic or additive activity.

## Claims

1. A TIGIT polypeptide variant, comprising a TIGIT extracellular domain or a functional fragment thereof, **characterized in that**, relative to the amino acid sequence SEQ ID NO. 3, the variant comprises at least one amino acid mutation, the mutation position being selected from one or more of the following group: residues I42, Q56, N70, L73, G74, Y113.

2. The TIGIT polypeptide variant according to claim 1, **characterized in that** the amino acid mutation is an amino acid substitution.

3. The TIGIT polypeptide variant according to claim 1 or 2, **characterized in that** the amino acid mutation is selected from one or more of the following group: I42V, I42L, Q56N, Q56E, N70Q, N70H, L73I, L73V, G74S, Y113W, Y113T, Y113F.

4. The TIGIT polypeptide variant according to any one of claims 1-3, **characterized in that** it comprises the amino acid sequence SEQ ID NO. 21 or an amino acid sequence having at least 95%, 96%, 97%, 98%, or 99% sequence homology with the amino acid sequence SEQ ID NO. 21.

5. A fusion protein comprising the TIGIT polypeptide variant according to any one of claims 1-4.

6. The fusion protein according to claim 5, **characterized in that** it further comprises (a) a CTLA4 polypeptide or a variant thereof, and/or b) an Fc polypeptide or a variant thereof, wherein the CTLA4 polypeptide comprises an extracellular domain or a functional fragment thereof.

7. The fusion protein according to claim 6, **characterized in that** the CTLA4 polypeptide variant comprises the A30Y and/or L105E amino acid mutations relative to the amino acid sequence SEQ ID NO. 1.

8. The fusion protein according to claim 6 or 7, **characterized in that** the CTLA4 polypeptide or variant thereof comprises an amino acid sequence selected from:
a) SEQ ID NO. 1;
b) SEQ ID NO. 9;
c) an amino acid sequence having at least 95%, 96%, 97%, 98%, or 99% sequence homology with SEQ ID NO: 1 or SEQ ID NO: 9.

9. The fusion protein according to claim 6, **characterized in that** the Fc polypeptide is a monomer or dimer of a human IgG1, IgG2, or IgG4 Fc polypeptide.

10. The fusion protein according to claim 6, **characterized in that** the Fc polypeptide comprises a hinge region, a CH2 domain, and a CH3 domain, or a CH2 domain and a CH3 domain; preferably, the Fc polypeptide variant comprises an amino acid mutation that improves Fc stability or half-life; preferably, comprises one or more amino acid mutations selected from the group consisting of: M428L, N434S, M252Y, S254T, T256E, IgG4 (S228P); more preferably, the IgG1 Fc polypeptide variant comprises the M428L, N434S or M428L/N434S, M252Y, S254T, T256E or M252Y/S254T/T256E amino acid mutations; the IgG4 Fc polypeptide variant comprises the S228P, M428L, N434S, M428L/N434S, M428L/S228P, N434S/S228P, M428L/N434S/S228P, M252Y/S228P, S254T/S228P, T256E/S228P or M252Y/S254T/T256E/S228P amino acid mutations.

11. The fusion protein according to claim 6 or 10, **characterized in that** the Fc polypeptide or variant thereof comprises an amino acid sequence selected from:
a) SEQ ID NO. 7;
b) SEQ ID NO. 8;
c) an amino acid sequence having at least 95%, 96%, 97%, 98%, or 99% sequence homology with SEQ ID NO: 7 or SEQ ID NO: 8.

12. The fusion protein according to claim 6, **characterized in that** the fusion protein comprises a polypeptide monomer selected from the following or a homodimer or heterodimer thereof:
a)A-L-Fc-L-B;
b)B-L-Fc-L-A;
c)A-L-B-L-Fc;
d)B-L-A-L-Fc;
e) Fc -L-A-L-B;
f) Fc -L-B-L-A;
wherein A is a TIGIT polypeptide variant, B is a CTLA4 polypeptide or a variant thereof, and L is absent or is a linker; preferably, the linker is (G4S)n or (SG4)m, wherein n or m is a positive integer selected from 0, 1, 2, 3, 4, 5, or 6.

13. The fusion protein according to claim 5, **characterized in that** the fusion protein comprises an amino acid sequence selected from SEQ ID NO. 23, SEQ ID NO. 25, or SEQ ID NO. 29, or an amino acid sequence having at least 95%, 96%, 97%, 98%, or 99% sequence homology therewith.

14. The fusion protein according to claim 5, **characterized in that** the fusion protein comprises the amino acid sequence of SEQ ID NO. 27, or an amino acid sequence having at least 95%, 96%, 97%, 98%, 99%, or 99% sequence homology therewith.

15. A nucleic acid, **characterized in that** it encodes the polypeptide variant according to any one of claims 1-4 or the fusion protein according to any one of claims 5-14.

16. An expression vector, **characterized in that** it comprises the nucleic acid according to claim 15.

17. A host cell, **characterized in that** it comprises the expression vector according to claim 16 or expresses the polypeptide variant according to any one of claims 1-4 or the fusion protein according to any one of claims 5-14.

18. A method for preparing the polypeptide variant according to any one of claims 1-4 or the fusion protein according to any one of claims 5-14, **characterized in that** it comprises the steps of: synthesizing the polypeptide variant according to any one of claims 1-4 or the fusion protein according to any one of claims 5-14, and/or, culturing the host cell according to claim 17 under conditions that express the polypeptide variant according to any one of claims 1-4 or the fusion protein according to any one of claims 5-14.

19. A pharmaceutical composition, **characterized in that** it comprises the polypeptide variant according to any one of claims 1-4, the fusion protein according to any one of claims 5-14, and optionally a pharmaceutically acceptable excipient or carrier.

20. Use of the polypeptide variant according to any one of claims 1-4, the fusion protein according to any one of claims 5-14, or the pharmaceutical composition according to claim 19 in the preparation of a medicament for the prevention or treatment of an immune system disease or tumor; preferably, the immune system disease comprises lupus erythematosus, lupus nephritis, Hashimoto's thyroiditis, diabetes, myasthenia gravis, pemphigus, multiple sclerosis, autoimmune hemolytic anemia, idiopathic thrombocytopenia, chronic active hepatitis, ulcerative colitis, rheumatism, arthritis, psoriasis, T cell lymphoma, autoimmune graft-versus-host disease (GVHD), organ transplant rejection.

21. The method according to claim 20, **characterized in that** the diabetes is type 1 diabetes.

22. The method according to claim 20, **characterized in that** the rheumatism or arthritis is rheumatoid arthritis.

23. A method for preventing or treating an immune system disease or tumor, comprising administering to a subject a pharmaceutically effective amount of the polypeptide variant according to any one of claims 1-4, the fusion protein according to any one of claims 5-14, or the pharmaceutical composition according to claim 19.

24. The method according to claim 23, **characterized in that** the immune system disease comprises lupus erythematosus, lupus nephritis, Hashimoto's thyroiditis, diabetes, myasthenia gravis, pemphigus, multiple sclerosis, autoimmune hemolytic anemia, idiopathic thrombocytopenia, chronic active hepatitis, ulcerative colitis, rheumatism, arthritis, psoriasis, T cell lymphoma, autoimmune graft-versus-host disease (GVHD).

25. The method according to claim 24, **characterized in that** the diabetes is type 1 diabetes.

26. The method according to claim 24, **characterized in that** the rheumatism or arthritis is rheumatoid arthritis.

27. The method according to any one of claims 23-26, **characterized in that** the method further comprises administering a second active molecule, wherein the second active molecule comprises an immunosuppressant, an immunomodulator, or an anti-inflammatory drug.

28. A fusion protein, **characterized in that** it comprises a first structural region and a second structural region, or comprises a first structural region, a second structural region, and a third structural region; the first structural region is a TIGIT polypeptide or a variant thereof, the second structural region is a CTLA4 polypeptide or a variant thereof, and the third structural region is an Fc polypeptide or a variant thereof; the TIGIT or CTLA4 comprises an extracellular domain or a functional fragment thereof.
